# EUROPEAN PATENT SPECIFICATION

(11) **EP 0 828 852 B1**
(45) Date of publication and mention of the grant of the patent: **25.06.2003**
(21) Application number: 95916864.2
(22) Date of filing: 10.05.1995
(51) Int. Cl.: C12Q 1/68, C07H 21/04, C12P 19/34, C12N 15/12, A61K 48/00, A61K 38/17, A61K 31/70, C07K 14/47, C07K 16/30, G01N 33/574

(54) **METHODS FOR DETECTION AND THERAPY OF SQUAMOUS CELL CARCINOMA AND BLADDER CARCINOMA, IN PARTICULAR FOR DETERMINING THE PRESENCE AND TREATMENT OF MINIMAL RESIDUAL DISEASE, MICROMETASTASES OR DISSEMINATION OF SUCH CARCINOMA TYPES**
VERFAHREN ZUM NACHWEIS UND THERAPIE VON SCHUPPENARTIGEN ZELLKARZINOMEN UND BLASEN-KARZINOMEN INSBESONDERE ZUR BESTIMMUNG DER ANWESENHEIT UND BEHANDLUNG VON MINIMALEN RESTERKRANKUNGEN, MICROMETASTASIERUNG ODER DER VERBREITUNG VON SOLCHEN KARZINOMTYPEN
PROCEDES POUR LA DETECTION ET LA THERAPIE DE L'EPITHELOMIA EPIDERMOIDE, DU CANCER DE LA VESSIE, EN PARTICULIER, POUR DETERMINER LA PRESENCE ET LE TRAITEMENT DE LA MALADIE RESIDUELLE IMPERCEPTIBLE, DES MICROMETASTASES OU LA DISSEMINATION DE CE TYPE DE CARCINOMES

(43) Date of publication of application: 18.03.1998
(73) Proprietor: Stichting Keel-, Neus en Oorheelkunde, 1066 CX Amsterdam (NL)
(72) Inventor: BRAKENHOFF, Rudolf, Henrikus, NL-3524 BJ Utrecht (NL); VAN DONGEN, Augustina, Antonius, Maria, Sylvester, NL-3572 ES Utrecht (NL)
(74) Representative: van Westenbrugge, André
(86) International application number: NL9500168
(87) International publication number: WO96035808

(56) References cited:
- AMERICAN JOURNAL OF PATHOLOGY, vol. 136, no. 1, January 1990 pages 191-197, QUAK, J. ET AL. 'A 22-Kd surface antigen detected by monoclonal antibody E48 is exclusively expressed in stratified squamous and transitional epithelia'
- ORAL ONCOLOGY, EUR. JOURNAL OF CANCER, vol. 30b, no. 2, January 1994 pages 82-87, GERRETSEN, M. ET AL. 'The feasibility of radioimmunotheraphy of head and neck cancers'
- ANTICANCER RESEARCH, vol. 13, 1993 pages 2533-40, QUAK, J. ET AL 'Perspectives of monoclonal antibodies for the detection and treatment of head and neck tumours'
- JOURNAL OF IMMUNOLOGY, vol. 8 , October 1992 pages 2615-18, GUMLEY, P. ET AL 'Isolation and characterization of cDNA clones for the mouse Thymocyte B Cell Antigen (ThB)' cited in the application
- PROC. NATL. ACAD. SCI. USA, vol. 90, - December 1993 pages 11478-82, FYNAN, E. ET AL 'DNA vaccines: Protective immunizations by parenteral, mucosal, and gene-gun inoculations' cited in the application
- PROC. NATL. ACAD. SCI. USA, vol. 90, December 1993 pages 11483-87, SHEN, Z. ET AL 'Identification of a cytochrome P450 gene by reverse transcription using degenerate primers containing inosine'
- JOURNAL OF CELL BIOLOGY, vol. 129, no. 6, June 1995 pages 1677-89, BRAKENHOFF, R. ET AL. 'The human E48 antigen, highly homologous to the murine Ly-6 antigen ThB, is a GPI-anchored molecule apparently involved in keratinocyte cell-cell adhesion. '

## Description

### Field of the invention

The subject invention lies in the field of malignant diseases. More specifically in the field of squamous cell carcinoma and bladder carcinoma. The invention covers diagnosis and treatment of such forms of carcinoma. In particular the determination of the presence of and treatment of minimal residual disease, micrometastases or dissemination of such carcinoma types as well as the means to be applied in such methods like the encoding nucleic acid sequences, constructs in expression vectors, proteins/peptides comprising epitopes and specific antibodies are described.

### Background of the invention.

### SQUAMOUS CELL CARCINOMA

Squamous cell carcinoma is the major histological type of tumors arising from the head and neck. lung, esophagus, cervix, skin and vulva. The potential impact of the invention on diagnosis and treatment of this tumor type will be mainly illustrated for squamous cell carcinoma of the head and neck (HNSCC) but is as important for squamous cell carcinoma of other origin. Head and neck cancer traditionally refers to malignant tumors that arise in the mucosa of the upper aerodigestive tract including the oral cavity, pharynx, larynx, nasal cavity, and paranasal sinuses. More than 90% of these tumors are squamous cell carcinomas¹.

Worldwide, more than 500.000 new cases of HNSCC are projected annually, and this incidence is rising ^{2,3}. HNSCC accounts for approximately 5% of all newly diagnosed malignant neoplasms in North Western Europe and the United States ^{1,2,4,5}. During 1994 approximately 42.100 U.S.A. citizens developed head and neck cancer and 11,725 died from head and neck cancer ². About one-third of the patients presents early stage disease (stage I and II), while two-third presents advanced disease (stage III and IV).

Spread of HNSCC at the primary site is dictated by the local anatomy, and each anatomic site has its own peculiar spread pattern ^{7,8}. The most important mechanism in the spread of HNSCC is by lymphatic dissemination. After local proliferation and infiltration into the lymph node, the capsule of the lymph node can ultimately be destroyed. A tumor infiltrated lymph node itself can act as a focus for further dissemination into the surrounding lymphatics or the blood stream ⁹. The incidence of lymph node involvement depends mainly on the site and size of the primary tumor. The status of the lymph nodes in the neck is one of the most important prognostic factors in head and neck cancer ¹⁰⁻¹³. The presence of lymph node metastasis in the neck decreases the expected survival with 50%. In addition, among patients with involved nodes, survival is significantly lower if more nodes are involved ¹⁴⁻²⁰, if there is nodal involvement at multiple levels ¹⁸, or if extranodal spread is present ^{15,16,21-24}. Survival worsens as lower lymph node levels are involved ²⁵⁻²⁷.

Distant metastases usually occur late in the disease. The overall incidence of clinical distant metastases in HNSCC is 10-24% ^{14,28-30}.

The actual incidence may even be higher as autopsy studies report an incidence of 37-47% ³¹⁻³³. Eighty percent of the distant metastases become manifest within 2 years after the appearance of lymph node metastasis ^{14,28}. The lungs are the most frequent initial site of distant metastases (52-60%), followed by the skeletal system (19-35%). Other localizations of metastatic deposits are the liver, mediastinum, skin, and brain ^{14,28,34}. The rate of distant metastases correlates more with the appearance of cervical node metastases than with the primary tumor stage ^{15,28,29,35}. The number of histopathologically positive lymph nodes, extra nodal spread, and lymph node metastases at multiple levels in the neck have been shown to be risk factors for the development of distant metastases ^{14,15,30,35}. Patients with more than three lymph node metastases have a risk of almost 50% to develop distant metastases ¹⁴.

Because HNSCC arises from mucosal surfaces, most primary tumors can be detected by physical examination. In almost all patients an endoscopic evaluation is performed. The major objectives of endoscopy are confirmation of the diagnosis by biopsy and determination of the extent of the lesion ^{36,37}. The tumor stage can be assessed more accurately if endoscopic findings are used in conjunction with imaging techniques, like computed tomography (CT) and magnetic resonance imaging (MRI) ³⁸⁻⁴⁰. The assessment of the status of the neck lymph nodes is essential for optimal treatment planning. In many institutions, this is mainly based on palpation. However, palpation is not reliable for the detection of lymph node metastasis ^{25,40,41}. Histopathological studies have demonstrated that both the false positive rate and the false negative rate are unsatisfactory high ^{42,43}. The overall error of palpation is about 20-30% ^{26,41,42,44}. Modern imaging techniques, like CT, MRI, ultrasound (US), and US-guided fine needle aspiration cytology (FNAC) are more reliable than palpation, but still leave much to be desired ⁴⁴⁻⁵¹. The capability of all the above mentioned techniques to detect small tumor deposits is limited. Moreover, these imaging techniques very often cannot discriminate between normal lymph nodes, reactively enlarged lymph nodes, and tumor infiltrated lymph nodes. Because of these problems with the pretreatment assessment of the status of the neck, it may be difficult to decide whether the neck should be treated or not, thus resulting in over- and undertreatment of the neck. Moreover, these conventional imaging techniques provide only locoregional information, while no information on systemic dissemination is obtained, whilst the presence of distant metastases has an important impact on the choice of the treatment. In conclusion, there is a great demand for a specific method to detect small metastases/disseminated tumor cells in the lymph nodes in the neck as well as at distant sites.

The review article¹³⁴ by E. Vokes et al provides an overview of the field of head and neck cancers and illustrates the serious problems related to carcinogenesis diagnosis and therapy that still exist notwithstanding the amount of time, effort and money invested in research. Information regarding the genetic basis of the multistep process of carcinogenesis is accumulating and a number of chromosomal deletions and rearrangements have been identified. By way of example we name molecular changes of epidermal growth factor receptor and p53, amplification of int-2 and bcl-1 and deletion of chromosome 18q as well as suggestion of effects on other tumor suppressor genes. Phenotypic and immunological changes have also been signalled, although it is unclear whether these changes are intrinsic to the disease, the host or the extent of the disease.

By way of example of phenotypic disease the above cited article mentions that alterations in differentiation antigens including cytokeratins, cornified envelope proteins, blood group proteins and other glycoproteins occurs. It also reports that immunologic alterations appear to affect both cell mediated and humoral immunity.

Therapy usually consists of surgery, radiotherapy, or a combination of these modalities. Generally, patients with stage I and II disease undergo either surgery or radiotherapy with curative intent⁵². This goal will be achieved in 80% of the patients with stage I disease and in more than 60% of patients with stage II disease. For patients with local or regional advanced disease (stage III and IV) the prognosis is much worse. Frequently, a planned combination of surgery and radiotherapy is used to treat these patients^{53,54}, but cures are only seen in less than 30% of cases⁵⁵. It is disappointing that the survival rates of patients with advanced stage head and neck cancer have not increased much over the last decades². Although many studies have observed a decrease of local and particularly regional failures by the widespread use of planned combined surgery and radiation therapy ^{13,17}, this has not been reflected in a proportional increase of the five-year survival rates. As fewer patients die from uncontrolled disease in the head and neck, more are exposed to the risk of disseminated disease below the clavicles ^{56,57}. It is obvious that there is a great demand for a systemic treatment effective in destroying micrometastases at distant sites and minimal residual disease at the local or regional level. Initially hopes were high for chemotherapy to play this role, but unfortunately these expectations have not been fulfilled ^{34,58-60}.

To date metastatic disease is usually incurable and the use of chemotherapy is generally directed at reducing symptoms to improve quality and length of life. Metastatic or recurrent disease is the single commonly accepted indication for chemotherapy as standard therapy. Active single agents include methotrexate, cisplatin, carboplatin, infusional fluorouracil, hydroxyurea, doxorubicin, bleomycin, ifosfamide, edatrexate and taxol. However rates of response are 30% or less and response lasts only 2-6 months. Combination chemotherapy has resulted in slightly better results with regard to response rates but has not been very successful with regard to prolonged survival.

The problem of detection of metastases in general has been addressed in a large number of publications, however the specific problem for solid head and neck tumors has not yet been successfully addressed. In fact a number of the general measures proposed have been illustrated in retrospect not to be effective in practice. Clearly, a more tumor specific systemic therapy is needed.

Preceding 1991 a large number of groups published work suggesting linking CD44 expression to cell adhesion for leukocytes, bone marrow cells and several epithelial cell types for example colonic carcinomas which apparently expressed more than normal colonic epithelium¹³⁵. Birch M. et al¹³⁶ reported that melanoma cell lines recognised by an anti CD44 antibody were more metastatic than clones which weakly expressed CD44. Gunthert U. et al.¹³⁷ linked metastatic ability of rat pancreatic adenocarcinoma cells to CD44 expression and Sy M.S. et al.¹³⁸ did the same for human lymphoma cells. In WO 91/17248 variant CD44 proteins and use thereof in diagnosis and therapy were claimed. Three groups also published work regarding the absence of ability of linking CD44 expression to metastatic capability using PCR. (Hofmann M. et al; Stamenkovic I. et al.; Jackson D.G. et al)¹³⁹⁻¹⁴¹

In WO 94/02633 and WO 94/12631 it was disclosed that marked overexpression of multiple splice variants of the CD44 gene occurs in tumour tissue in comparison to normal tissue. This observation thus should according to the inventors (Tarin D. and Matsumara Y.) form the basis of a method of diagnosing neoplasia by analysis of a sample of body tissue or body fluid or waste product. Examples of samples that could be used are body tissue or body fluid as opposed to cells cultured in vitro. They have claimed a new exon 6 of the CD44 encoding gene as such and for use in the diagnostic method as their results illustrated a clear difference in CD44 expression in metastatic (malignant) tumors, non metastatic locally invasive tumors and benign tumors as well as normal tissue. They have also claimed the use of the peptide corresponding to CD44 exon 6 and antibodies specific for said peptide as such and for diagnosis of tumors. Their studies were directed at tumors of the breast and colon. They were not directed at carcinomas of the squamous cell type. They also indicate it is not expected that further research will reveal that the natural (non mutated) products of any individual exon of CD44 will be uniquely present in tumor cells and not in their normal counterparts but that an abnormal pattern of expression of the CD44 locus could be the cause of malignancy or the result of other genetic disturbances causing such a disturbance.

Already in 1990 Schlimok G. et al.¹⁴² described the use of a monoclonal antibody specific for polypeptide cytokeratin 18 specifically expressed in cells derived from simple epithelia for detection of epithelial tumor cells in bone marrow aspirates. Clinical follow-up studies showed a significantly higher relapse rate in patients presenting CK18 positive cells in their bone marrow at the time of their surgery. As to the critical issue of specificity anti CK antibodies appeared to be superior to antibodies against the epithelial membrane antigen (EMA), which is also found on cells of the lymphoid lineage or to the 17-1A antigen which is absent from variable proportions of tumor cells¹⁴². This was the first article to present data on bone marrow micrometastases in colorectal cancer, where in contrast to breast cancer, overt bone or skeleton metastases are rare. It came as a surprise considering the low clinical incidence that a high frequency of detection of bone marrow metastases was found.

Also in 1990 Schlimok G. and Riethmuller G. disclosed in Seminars in Cancer Biology¹⁴³ that monoclonal antibodies had been used to detect tumor cells in bone marrow of patients with neuroblastoma, breast cancer, small cell lung cancer, prostate cancer and gastrointestinal cancers. Immunocytology proved to be more sensitive than conventional cytology and histology and had the additional advantage of specificity. The proliferative potential of micrometastatic cells was assessed by characterisation of EGF and transferrin receptors. Follow-up studies indicated that the presence of disseminated tumor cells in bone marrow can be taken as predicting the subsequent development of overt metastasis. The most critical factor according to the authors of this article are that the antibody should fulfil two conditions 1) it should recognise an antigen expressed in or on all tumor cells and 2) it should not react with haematopoietic or other cells of the bone marrow. Since under normal circumstances epithelial cells are not present in bone marrow, antibodies specific for epithelial antigens could be used. That the selection of a suitable antibody is however far from simple is illustrated by the fact that antigens HMFG1, HMFG2 and EMA known to be widely distributed in epithelial tissues and shown to be expressed on epithelial tumor cells in bone marrow exhibit extensive cross reaction with epitopes on normal bone marrow cells. In addition to the cell membrane markers the family of cytokeratin type were considered useful as markers of cells of epithelial origin. They are not tumor specific and are present on both malignant and non malignant cell types. However a caveat was issued that uncoordinated expression of cytokeratin components 8 and 18 could occur in some non-epithelial cells.

In The Lancet 1991; Smith B. et al¹⁴⁴ disclose the use of PCR to make complementary DNA from peripheral blood messenger RNA to amplify cDNA specific for a gene actively transcribed only in the tumor tissue type. PCR was used to amplify the gene for tyrosinase a tissue-specific gene in melanocytes. Since normal melanocytes were not thought to circulate in peripheral blood, detection of tyrosinase transcription in normal blood should indicate the presence of circulating cancer cells. This method for melanocyte detection was found to be extremely sensitive and could detect a single melanoma cell from a cell line in 2 ml of normal blood. The encouraging results suggested that the method could be clinically very useful. The method takes only 2-3 days and thus could be performed routinely. The article furthermore states it is not clear whether the method would be applicable in patients with apparently localised disease in whom tumor cells in peripheral blood may be few or absent. The article also discloses that only extensive prognostic evaluation will determine whether the presence of tumor cells in the peripheral blood is always ultimately associated with metastases or whether implantation need not be inevitable.

The diagnosis of naematogenous micrometastasis by RT-PCR in patients with prostate cancer was reported¹⁴⁵ by Moreno J.G. et al. as being more sensitive than immunohistochemistry in detecting micrometastatic prostate cancer cells. RT-PCR with a primer set amplifying the cDNA encoding a prostate specific antigen, which is exclusively expressed by prostatic epithelial cells, was used to detect circulating prostatic cells. The entire PSA-RNA assay consisted of four steps a) isolation of nucleated cells from whole blood by gradient centrifugation, b) extraction of total RNA, c) RT-PCR on total RNA with PSA specific primers and d) agarose gel electrophoresis of PCR products. Primers had to be selected that were a) specific to the PSA gene mRNA, b) did not react with the HMGK gene which is highly homologous to PSA (75-85%) and c) were spanning an intron to prevent amplification of genomic DNA sequences, always present in RNA preparations. A number of problems were, however, still apparent as the prostate cells may be more concentrated in the buffy coat and circulating prostate cancer clones could be more poorly differentiated thereby expressing lower levels of PSA. The use of other markers that may be less influenced by malignant dedifferentiation such as cytokeratin 19 is suggested in this article.

In 1994 Wollenberg, B. et al.¹⁴⁶ suggested the application of monoclonal antibodies raised against cytokeratin 19 for detecting individual disseminated epithelial tumor cells. Cytokeratin 19 expressing cells were not found in the bone marrrow of 18 patients with non-malignant disease. Of 46 patients initially exhibiting no tumour cells in the bone marrow only 14 had a clinical recurrence whereas 17 of 27 patients exhibiting the presence of epithelial cells at the time of primary treatment appeared to indicate a significantly higher risk of development of local or distant tumor recurrences. The finding of occult tumour cells in the bone marrow was therefore suggested to be an independent significant prognostic variable of a late clinical relapse. The Wollenberg method, however, fails on samples like blood because these samples exhibit false positives. This could be attributed to the lack of specificity of cytokeratin for epithelial cells, the presence of epithelial cells in a blood sample or experimental artefacts of the staining procedure. The second point would render application of an analogous technique with a different marker specific for an epithelial cell useless. The former would potentially leave the option open of using a different marker which really is specific for epithelial cells, however a marker with such specificity is not known to date.

In summary, a number of methods using cell-specific markers for the detection of specific cell types in body samples not usually carrying such cells have been suggested. The difficulties and problems that can be encountered when trying to implement such a method can be summarized repectively ranked. Firstly a typically cell-specific marker is required and must be found, secondly the cell type to be detected must not normally be present in the sample, thirdly the sample must be a tissue that can contain the specific cell type i.e. the tumor cell, fourthly the marker must be expressed by the cell type to be detected in the sample. fifthly, the method of detection should be sensitive and reliable, preferably if possible capable of detecting a single cell. Alternatively, the method can comprise detection of expression of a product which is specific for any type of metastatic cell i.e. a product actually involved in the process of metastasis or linked to such a product, wherein the difficulty lies in finding a general metastasis-specific expression product. Thusfar it has not been possible to detect metastasis of squamous cell type or bladder type in blood samples in a specific and sensitive manner without large numbers of false positives as no marker exhibiting the required characteristics has been taught or suggested and no general marker of metastasis is available.

Several suggestions of markers for squamous differentiation have been given. Examples hereof are involucrin¹⁴⁷ and keratin 10¹⁴⁸. These markers, however, do not fulfil the required criteria and thus cannot be used to detect squamous cell carcinoma cells.

In 1990 Quak J.J. et al.¹⁰⁸ described a monoclonal antibody (MAb) that recognises squamous epithelial cells. The antibody was raised against a metastasis of a moderately differentiated squamous cell carcinoma. Most squamous epithelial cells of oral mucosa reacted with the E48 antibody. Similar staining was observed with stratified epithelia of the esophagus, the vagina and human epidermis. In epidermis no staining was seen in the stratum corneum. Sweat and sebacous glands were negative whereas the outer root sheath of a hair follicle did react. Among the non-neoplastic tissues tested further only transitional epithelial cells of the urinary bladder reacted. No bone marrow or blood samples were taught or suggested as samples to be considered for detection of antigen specific for the MAb E48. All that is taught concerning the antigen is that it was a single band with molecular weight 22.000 under non-reducing conditions which comprised an epitope for MAb E48 that was sensitive to reduction. It was indicated further that the antigen is an unknown human antigen. It was also suggested that it could be a suitable target for immunotargeting in vivo. Neither the antibody nor the antigen have been indicated as useful for the detection of micrometastases of squamous cell carcinoma or bladder carcinoma.

The monoclonal antibody E48 (MAb E48) has subsequently been tested extensively in *in vivo* model systems as well as in radioimmuno-scintigraphy studies in patients with squamous cell carcinoma. Biodistribution and dosimetry calculations indicated the feasibility of adjuvant radioimmunotherapy for the eradication of minimal residual disease after initial surgical treatment^{77,69,70}.

Others ¹⁵⁶ suggested that Mab E48 antibody could perhaps be capable of interfering with tumour cell-cell contacts in such a way that this may prevent the formation of new colonies (metastases) at moments when single tumours cells get access to the circulation e.g. during surgery. However, nothing was taught or suggested with regard to the characterisation of the antigen of the Mab E48 antibody.

There was some research carried out in order to functionally characterise the target antigen. It was determined that the antigen has a molecular weight between 15-20 kDa and *in vitro* experiments provided evidence that the antigen is involved in the (desmosomal) adhesion of keratinocytes¹¹⁴.

Desmosal adhesion is only one of the various filament anchorage systems of cellular adhesion that exist in a particular type of epithelial cell. Among the adhering junctions which require calcium ions for functioning, two major categories can be distinguished. One category consists of junctions attached to the microfilaments of the cytoskeleton (actin). These junctions are composed of a transmembrane complex, containing adhesion molecules of the cadherin family, and a plaque structure formed by the cytoplasmic domain of the cadherins and a complex set of associated proteins. In the E-cadherin complex, the actual adhesion molecule E-cadherin, a 120-140 kD transmembrane protein, is associated with plakoglobin. a structural component of all calcium dependent junctions, and the α- and β-catenins (γ-catenin is thought to be identical to plakoglobin) which are thought to be involved in the structural and functional interactions with for instance the cytoskeleton^{123,87}.

Besides their function in cell adhesion, the junctions are involved in signal transduction processes implicated in alterations of cell density, loss of matrix adherence etc., and can in this way influence cell behavior and morphology^{127,129,113}. The interaction of protein kinase C and protein kinases of the src family with E-cadherin adhesion junctions, has been established by several lines of evidence, and these molecules may well be involved in the signal transduction pathways^{126,87}. Moreover, the role of E-cadherin junctions in cellular growth has been determined *in vitro* as well as *in vivo*, and one of the proteins associated with the junctions. β-catenin interacts with the gene product of the APC tumor suppressor gene, a gene in which loss of function mutations are involved in the development of epithelial tumors¹²¹.

The second category of cellular junctions consists of desmosomes and hemidesmosomes, the attachment sites for the intermediate filaments: the cytokeratins in epithelial cells and desmin and vimentin in most other cells. The desmosomes contain the cadherins desmocollin and desmoglein, which form the actual cellular connection, as well as the plaque proteins plakoglobin, desmoplakin and several other cell-type specific components^{116,125} The involvement of these second category junctions on the behavior of the cell is less well defined. Keratinocytes of squamous epithelia are attached to their adjacent cells by large numbers of desmosomes, and it seems possible that these cellular organelles may play important roles in the development of transformed cells to invading and metastasic tumor cells, however knowledge of this kind is still scarce.

Although a large number of the desmosomal constituents have been characterized, and the encoding genes have been cloned, some of the components are still unidentified, such as the small 22 kDa glycoprotein, formerly named desmoglein III or dg 4, which is co-purified in desmosomal preparations of bovine muzzle^{88,116}. The restricted pattern of expression and additional experimental data suggested that the MAb defined E48 antigen is identical to that small protein formerly referred to as desmoglein III/dg 4. Immunoelectronmicroscopical analysis with MAbE-48 on squamous tissue sections showed that a considerable amount of antigen is located in the midline of the desmosomes, indicating a putative involvement in desmosome formation.

### Detailed description of the invention

The subject invention involves detection of squamous cell carcinoma and bladder carcinoma, in particular for determining the presence of and treatment of minimal residual disease, micrometastases or dissemination of such carcinoma types as well as the means to be applied in such methods like nucleic acid sequences, constructs in expression vectors, proteins or peptides comprising relevant epitopes and antibodies. The various aspects of the invention centre round elucidation of the identity of a marker that is specific for squamous cell carcinoma and bladder carcinoma and can be used for detection of such carcinoma in a body sample such as blood without getting false positive results. The antigen of the monoclonal antibody MAb E48 was selected from a number of potentially specific markers for squamous cells as a marker useful for such a method. Apart from uncertainty as to whether the marker would in fact function as such or what problems might be encountered similar or different to those already faced in the state of the art for analogous projects as described in the introduction the marker had to be identified and isolated which was by no means straightforward. This will be elucidated furtheron in the description.

Through determination of the family of proteins to which the antigen MAb E48 belongs and of the nature of the nucleic acid sequence and amino acid sequence of the antigen it has now become possible to develop tests for detection of squamous cell carcinoma and bladder carcinoma in a sample using nucleic acid technology. A technology that enables extremely low levels of detection, thus making it more sensitive than immunoassays.

A method for detection of squamous cell carcinoma and bladder carcinoma according to the invention comprises the detection of the expression of nucleic acid encoding a glycosyl-phosphatidylinositol-(=GPI)-anchored protein in a manner known per se for nucleic acid detection, wherein the GPI-anchored membrane protein belongs to the family of human proteins having a molecular weight below 30 kDa comprising a cysteine-rich amino acid sequence with 10 cysteines in a contiguous amino acid sequence of 138 amino acids or less, said family to be referred to as human cysteine-rich GPI-anchored proteins (=human CRG proteins). Said family of proteins to be detected preferably comprising the following amino acid consensus sequence CX₁₋₅CX₅₋₁₅CX₃₋₈CX₃₋₈CX₁₀₋₃₀CX₂₋₇CX₁₀₋₃₀CCX₁₋₈CN, more preferably CX₁₋₅CX₅₋₁₅CX₃₋₈CX₃₋₈CX₁₀₋₃₀CX₂₋₇CX₁₀₋₃₀CCX₁₋₆DLCN and most preferably CX₂CX₉CX₄ₒᵣ₅CX₆CX₁₂₋₂₂CX₃₋₅CX₁₆₋₁₉CCX₂₋₄DLCN, wherein X can be any amino acid, C is cysteine, D is aspartic acid, L is leucine and N is asparagine. In particular the consensus sequence can be CX₂CX₉CX₄ₒᵣ₅CX₆CX₁₂₋₂₂CX₃₋₅CX₁₆₋₁₉CCX₂DLCN and most preferably the consensus sequence will be CX₂CX₅CX₅CX₆CX₁₇CX₃CX₁₈CCX₂DLCN, wherein X can be any amino acid, C is cysteine, D is aspartic acid. L is leucine and N is asparagine. Particular embodiments of the consensus sequence that can be found comprise LX₁₋₃CX₁₋₅CX₅₋₁₅CX₃₋₈CX₃₋₈CX₁₀₋₃₀CX₂₋₇CX₁₀₋₃₀CCX₁₋₈CN, more preferably LX₁₋₃CX₁₋₅CX₅₋₁₅CX₃₋₈CX₃₋₈CX₁₀₋₃₀CX₂₋₇CX₁₀₋₃₀CCX₁₋₆DLCN, most preferably LXCX₂CX₉CX₄ₒᵣ₅CX₆CX₁₂₋₂₂CX₃₋₅CX₁₆₋₁₉CCX₂₋₄DLCN, in particular the consensus LXCX₂CX₉CX₄ₒᵣ₅CX₆CX₁₂₋₂₂CX₃₋₅CX₁₆₋₁₉CCX₂DLCN and most preferably the consensus LXCX₂CX₅CX₅CX₆CX₁₇CX₃CX₁₈CCX₂DLCN, wherein X can be any amino acid, C is cysteine, D is aspartic acid, L is leucine and N is asparagine. The consensus sequence comprises the extracellular membrane bound portion of a CRG protein and of particular interest is the consensus sequence of the E48 antigen. The sample to be used is preferably a human sample. Suitable samples are samples of tissue or body fluid. Bone marrow, lymph nodes and peripheral blood or serum samples are suitable embodiments of samples that can be analysed with the method according to the invention. A preference may exist for non-invasive samples in view of prevention of discomfort to the patient. Further examples comprise cervical scrapings, urine, sputum or stool. In general a sample to be used in the method according to the invention should be a sample normally free of mRNA encoding a hCRG protein i.e. a sample that does not normally express nucleic acid encoding the hCRG protein to be detected. Such a sample must normally be free of the mRNA encoding the nCRG protein to be detected to avoid detection of false positives. Suitable samples are samples normally free of keratinocytes or cells from transitional epithelium as only these do express the E48 antigen. In particular the method according to the invention can be used to analyse whether purged tissue is free of minimal residual disease, micrometastases or dissemination of the squamous carcinoma type or the bladder carcinoma type after having undergone purging for such carcinoma types. Such purged tissue can suitably be blood or bone marrow. Ex vivo methods of purging have been described by Cribben et al 1991 and Roy et al 1991. Such a method comprised for example purging with anti-CD33-ricin-toxin fusion proteins.

The E48 antigen encoding nucleic acid sequence has been found for humans to be located on chromosome 8. It is to be expected that the encoding sequences for a number of proteins are to be found on chromosome 8. The method according to the invention thus comprises detection of nucleic acid sequences being expressed that are encoded by a nucleic acid sequence located on chromosome 8 and more specifically in the 8q24-qter region. In particular the method according to the invention is directed at detection of nucleic acid encoding CRG proteins belonging to the family of human Ly-6 proteins. A protein belonging to the family of Ly-6 proteins that is preferred will exhibit more than 50% homology with the amino acid sequence of Sequence id no 2. More preferably the homology will be higher than 66%, preferably more than 80% and more particularly 30-100%.

A method according to the invention comprises detection of a nucleic acid sequence encoding the amino acid Sequence Id. No. 2 or the complementary sequence thereof. Such a sequence can be the nucleic acid sequence according to Sequence Id. No. 1. Alternatively the nucleic acid sequence to be determined can be a nucleic acid sequence capable of hybridising to any of the nucleic acid sequences of the two aforementioned groups under normal to stringent hybridisation conditions. Such hybridisation occurring in a manner known per se, for example using normal hybridisation condition, comprising hybridisation at T=55°C with 6xSSC or using stringent hybridisation conditions comprising hybridisation at T=65°C with 6xSSC. The rinsing steps usually carried out in hybridisation methods are critical and can suitably comprise rinsing at T=55°C with 6xSSC when using normal conditions or when using stringent hybridisation conditions comprising rinsing at T=65°C with 0,2xSSC at T=65 °C. The idea being that the method according to the invention covers detection of genetic variants of CRG encoding sequences, in particular the human variants. The sequences to be detected should preferably encode functionally active proteins. Specifically variants differing only in one to five amino acids from the sequence id no 2 are comprised within the scope of the invention. It is generally known to a person skilled in the art that alteration of one amino acid need not alter the function of the protein. Most particularly if said alteration is not located within the consensus sequence. A nucleic acid sequence to be detected may comprise a sequence exhibiting of at least 50% homology, preferably at least 66%, more preferably at least 70% with more preference for 80-100% with a sequence according to Sequence Id no 1 or the nucleic acid sequence to be detected may be the complementary nucleic acid sequence thereof. Most particularly the product encoded by a nucleic acid sequence to be detected will be a CRG protein and as such will be cleavable by phosphatidinylinositol-specific phospholipase C treatment thus offering an assay for determining the identity of the product encoded by a nucleic acid sequence to be detected according to the invention. Alternatively a nucleic acid sequence to be detected may encode a product exhibiting the biological activity of cell-cell/cell-matrix adhesion in particular upon expression in human squamous cells such as UM-SCC-22B. Proteins or polypeptides exhibiting the aforementioned characteristics may be considered markers specific for certain cell types.

In a specific embodiment the method comprises analysis of the sample for a CRG protein for example the E48 antigen comprising making cDNA from mRNA present in the sample, amplifying portions of the cDNA corresponding to the expression product to be determined or a part thereof and detecting the amplified cDNA. These steps are routine methods in the field of nucleic acid amplification and are often employed in diagnosis of other diseases nowadays. Examples in other fields of diagnosis are presented in references 144, 145, 149 and the methodology illustrated in these articles is hereby incorporated mutatis mutandis for the subject field of diagnosis.

The amplification of cDNA can for example be carried out using the Reverse Transcriptase Polymerase Chain Reaction (RT-PCR) or another commonly used amplification reaction such as NASBA or nested primer amplification. For amplification reactions one or more primers must be selected using the nucleic acid sequence encoding the CRG that is to be detected. The amplified part can comprise one or more exons. In order to distinguish the genomic amplification product of DNA contaminated in the RNA preparation from amplification product of mRNA it is useful to amplify nucleic acid material such that at least one intron excision site is included i.e. the amplified product comprises at least one exon-exon junction. In this way the product amplified from the mRNA will be shorter than that amplified from genomic nucleic acid or the genomic product may not be amplified if the intron is larger than 4 kb. By way of example a sense primer of exon 2 of the nucleic acid sequence id no 1 can be suitably used. Alternatively a sense primer of exon 1 of the nucleic acid sequence according to sequence id no 1 and 3 and an antisense primer of exon 2 or 3 of the nucleic acid sequence according to sequence id no 1 and 3 can be used in a method according to the invention. The selection of primers will determine the length of amplification product. There is no critical length, thus the selection of primers is relatively free. Naturally the amplified product must be sufficiently long to be detectable in a manner known per se.

The amplified cDNA can be separated by electrophoresis followed by Southern blotting, hybridisation and autoradiography. The electrophoresed amplified products can be blotted onto a nylon membrane, thereby immobilising them followed by probing with a probe capable of hybridising to or binding to the amplified product. There are also many alternatives for analysing the amplified product. In general terms the amplified product is to be detected using a molecule capable of specifically binding to a part of the nucleic acid sequence to be detected or to the complementary sequence thereof in a manner known per se for nucleic acid detection. The molecule can be a nucleic acid sequence of at least 10 nucleotides capable of specifically binding to a part of the nucleic acid sequence to be detected or the complementary sequence thereof under normal to stringent hybridisation conditions (see previously for further elucidation on the conditions or see instruction manuals of amplification kits). The product to be detected may optionally be immobilised prior to detection. The probe can be provided with a label that is detectable or can be detectable such as a radioisotope. fluorescent or chromophoric molecule or an enzyme. Suitable labels and probe types will be apparent to a person skilled in the art of detection of nucleic acid amplification products. Alternatively the probe for detecting may be immobilised and the amplified product that is bound subsequently to the probe can then be detected in a manner known per se for sandwich assays.

The invention is also directed at components to be used in a detection method of squamous cell type carcinoma and bladder type carcinoma. Firstly the invention thus covers a number of novel nucleic acid sequences as illustrated below.
- A nucleic acid sequence encoding the amino acid sequence of sequence id no 2 or being the complementary nucleic acid sequence thereof.
- A nucleic acid sequence according to sequence id no 1 or the complementary sequence thereof.
- A nucleic acid sequence capable of hybridising to a nucleic acid sequence belonging to the two aforementioned groups under normal to stringent hybridisation conditions, preferably stringent hybridisation conditions in a manner known per se, wherein normal hybridisation conditions comprise hybridisation at T=55 °C, with 6xSSC and wherein stringent hybridisation conditions comprise hybridisation at T=65 °C, with 6xSSC. Such a nucleic acid sequence is further characterised by the fact that the hybridisation conditions comprise rinsing with 6xSSC at 55 °C and 0.2xSSC at T=65 °C respectively.
- A nucleic acid sequence exhibiting at least 50% homology, preferably more than 66% with more preference for 80-100% with a nucleic acid sequence according to sequence id no 1 or the complementary sequence thereof.
- A nucleic acid sequence according to any of the above described categories encoding a product that can be cleaved by phosphatidylinositol-specific phospholipase C treatment.
- A nucleic acid sequence according to any of the above described categories encoding a product exhibiting biological activity of cell-cell/cell-matrix adhesion in particular upon expression in human UM-SCC-22B cells.
In short nucleic acid sequences encoding human CRG proteins and functional variants thereof fall within the scope of the invention.

The invention is also directed at a part of any of the nucleic acid sequences of the above described categories, said part being at least 10 nucleotides in length and having a length sufficient to use as probe or primer or said part being at least 21 nucleotides in length and having a length suitable for eliciting immune response which length will be apparent to a person skilled in the art. Preferably the part will comprise a part of the extracellular membrane bound portion of the CRG, wherein the CRG anchored membrane protein belongs to the family of human proteins having a molecular weight below 30 kDa comprising a cysteine-rich amino acid sequence with 10 cysteines in a contiguous amino acid sequence of 138 amino acids or less. Such a protein will comprise the consensus sequence CX₁₋₅CX₅₋₁₅CX₃₋₈CX₃₋₈CX₁₀₋₃₀CX₂₋₇CX₁₀₋₃₀CCX₁₋₈CN, more preferably CX₁₋₅CX₅₋₁₅CX₃₋₈CX₃₋₈CX₁₀₋₃₀CX₂₋₇CX₁₀₋₃₀CCX₁₋₆DLCN and most preferably CX₂CX₉CX₄ₒᵣ₅CX₆CX₁₂₋₂₂CX₃₋₅CX₁₆₋₁₉CCX₂₋₄DLCN, in particular the consensus CX₂CX₉CX₄ₒᵣ₅CX₆CX₁₂₋₂₂CX₃₋₅CX₁₆₋₁₉CCX₂DLCN and most preferably the consensus CX₂CX₅CX₅CX₆CX₁₇CX₃CX₁₈CCX₂DLCN, wherein X can be any amino acid, C is cysteine, D is aspartic acid, L is leucine and N is asparagine. In other embodiments more specifically the protein may have the consensus sequence LX₁₋₃CX₁₋₅CX₅₋₁₅CX₃₋₈CX₃₋₈CX₁₀₋₃₀CX₂₋₇CX₁₀₋₃₀CCX₁₋₈CN, more preferably LX₁₋₃CX₁₋ ₅CX₅₋₁₅CX₃₋₈CX₃₋₈CX₁₀₋₃₀CX₂₋₇CX₁₀₋₃₀CCX₁₋₆DLCN, most preferably LXCX₂CX₉CX₄ₒᵣ₅CX₆CX₁₂₋₂₂CX₃₋₅CX₁₆₋₁₉CCX₂₋₄DLCN, in particular the consensus LXCX₂CX₉CX₄ₒᵣ₅CX₆CX₁₂₋₂₂CX₃₋₅CX₁₆₋₁₉CCX₂DLCN and most preferably the consensus LXCX₂CX₅CX₅CX₆CX₁₇CX₃CX₁₈CCX₂DLCN, wherein X represents any amino acid, C represents cysteine, D represents aspartic acid, L represents leucine and N represents asparagine. The consensus sequence comprises the extracellular membrane bound portion of a CRG protein. For the E48 antigen for example the amino acid sequence 21-94 of sequence id no 2 comprises the extracellular membrane bound portion. A probe comprising a nucleic acid sequence specific for this amino acid sequence will be particularly useful as genetic variation is most likely to be minimal in this region and mutations in such a consensus sequence are not as likely here as in a region that has not been associated with the consensus sequence. By way of example nucleotides 85-304 of sequence id no 1 encode such an extracellular membrane bound portion. Parts of this sequence of at least 10 nucleotides may thus be used to probe for this or other such CRG encoding sequences in a manner known per se.

The invention is also directed at a pharmaceutical composition comprising a construct in an expression vector, suitable for expressing nucleic acid in the patient, preferably being suitable for humans, said expression vector expressing a nucleic acid sequence encoding a CRG protein belonging to any of the categories described above, in particular encoding the E48 antigen or a genetic variant thereof as active component in a pharmaceutically effective amount, a pharmaceutically acceptable carrier and optionally an adjuvant. The invention also covers the constructs in expression vectors as such. It is disclosed for example in ref. 150 by Fynan E.F. that plasmid DNA expressing influenza virus hemagglutinin proteins have been tested for their ability to raise protective immunity against lethal influenza challenges of the same subtype. Parenteral routes of inocculation including intramuscular or intravenous routes were successful. Successful mucosal routes of vaccination included DNA drops administered to the nares or trachea. In particular use of a gene gun delivering DNA coated beads was most efficient, requiring only 0.4 micrograms of DNA in two immunisations for a 95% protective immunisation in mice. Bakker et al¹⁵¹ have used peptide fragments for therapy of melanoma. They used melanoma associated antigen gp100.

In analogy the methods disclosed in the cited articles can be applied mutatis mutandis for introducing nucleic acid sequences encoding CRG or parts of CRG of the categories disclosed for treatment

Another article of interest¹⁵² by author Greenhalgh D.A. et al. discloses a number of various delivery systems to be used when the epidermis is the target of gene therapy. The use of specific epidermal promoters is also described as resulting in both high levels of expression and keratinocyte expression. With such an application in mind the promoter of the E48 encoding gene is also claimed to form part of the invention. A part of the nucleic acid sequence according to sequence id no 3, wherein the part comprises the promoter sequence of the gene encoding the CRG of amino acid sequence id no 2 is hereby also claimed. The promoter sequence is comprised on the fragment of nucleic acid sequence from nucleotide 1 of sequence id no 3 to the first nucleotide of exon no. 1. The part of the nucleic acid sequence according to sequence id no 3 between nucleotides 1 to 495 is hereby also claimed. This comprises the fragment from nucleotide number 1 to the ApaI site of sequence id no 3 at exon 1. The use of such a promoter for therapeutical purposes also is covered by the scope of the invention. The teaching of the cited article regarding construction of suitable vectors and application therof in gene therapy is hereby enclosed by reference and can be used mutatis mutandis by a person skilled in the art on the basis of the information given here.

Ex vivo or in situ tumor cell detection using monoclonal antibodies or conjugates of antibodies selectively reactive with the antigen are known in the art and the clinical value for similar assays has been illustrated for colon carcinoma for example by Lindemann et al¹⁵³ for the 17-1A antigen.

A method of obtaining antibody specific for a marker of squamous cell carcinoma and bladder cell carcinoma in a sample not normally comprising keratinocytes or cells from transitional epithelium, said method comprising isolating a CRG anchored membrane protein, various categories of such a protein having being described above and in the claims or producing such a protein or a polypeptide comprising an epitope of such a protein synthetically or through recombinant DNA technology for example using a nucleic acid sequence encoding CRG according to any of the categories described above and in the claims, followed by inserting the isolated or synthesized protein or the putative polypeptide in a test animal, removing subsequently produced antibody and optionally making a hybridoma producing a monoclonal antibody in a manner known per se is also an embodiment covered by the scope of the invention. All the aforementioned methods can be suitably carried out if the CRG membrane anchored protein has an amino acid sequence id no 2 or is a functional equivalent thereof.

Where in the above antibodies have been mentioned as forming suitable components to carry out various embodiments of the invention the skilled person will of course realise that any other specific protein capable of specifically recognising and binding the CRG protein of interest is intended also to be covered by the invention. On the basis of computer modelling it should be possible to develop synthetic specifically binding molecules for the E48 antigen as now its amino acid sequence has been determined. In particular as Mab E48 a molecule that can specifically recognise and bind the E48 antigen is also available.
More detailed description of certain aspects of the invention related to the nucleic acid sequences encoding the GPI proteins according to the consensus disclosed

### Isolation and sequencing of the E48 antigen

As a first step towards the molecular cloning of the E48 encoding cDNA and gene, the protein was isolated from xenograft extracts through affinity chromatography and SDS-PAGE. The isolation was hampered by the fact that the antigen was poorly soluble and tended to precipitate during the subsequent steps. Nevertheless with some difficulty finally an isolation procedure could be established, enabling the purification of the E48 antigen in sufficient amounts for micro-sequencing. Antigen preparations obtained by immunoaffinity-absorption and analyzed by SDS-PAGE. Western blotting on PVDF membranes and Amido Black staining appeared to contain about 20% E48 antigen and 5 major co-purifying proteins, which have not been identified further as yet. For amino acid sequencing the E48 antigen band was excised from the PVDF membrane. The following amino acid sequence of the aminoterminal end was determined: LR?HVVTSSSNNK. As cysteines cannot be sequenced regularly, a putative C on position 3 was filled in since it was observed that a specific amino acid could not be detected at this position. After a few amino acids the signal decreased abruptly, usually an indication of amino acid modifications. The elucidated amino acid sequence was not found in the databases. We did however discern some similarity of the sequence with the mouse ThB antigen, a 15-20 kDa protein on the surface of murine lymphocytes. The homology found was as follows: 
hE48    L R ? H V V T S S S N N K
        * *   * *   *   *   *   *
mThB    L R C H V C T N S A N C K
 Based on this sequence various sets of degenerated primers were synthesized. The primers were used in RT-PCR reactions, hybridization. PCR on keratinocyte lambda gt11 libraries (Clontech; Westburg) etc. (Sambrook et al., 1989). PCR fragments were hybridized with both primers and cloned in pUC19. Clones containing both primers and with an insert of at least 400 bp were partially sequenced. Only 6 clones contained an open reading frame. They were all unknown in the databases, but none hybridized specifically with RNA isolated from squamous cell lines (data not shown).

### Molecular cloning of the E48 encoding cDNA

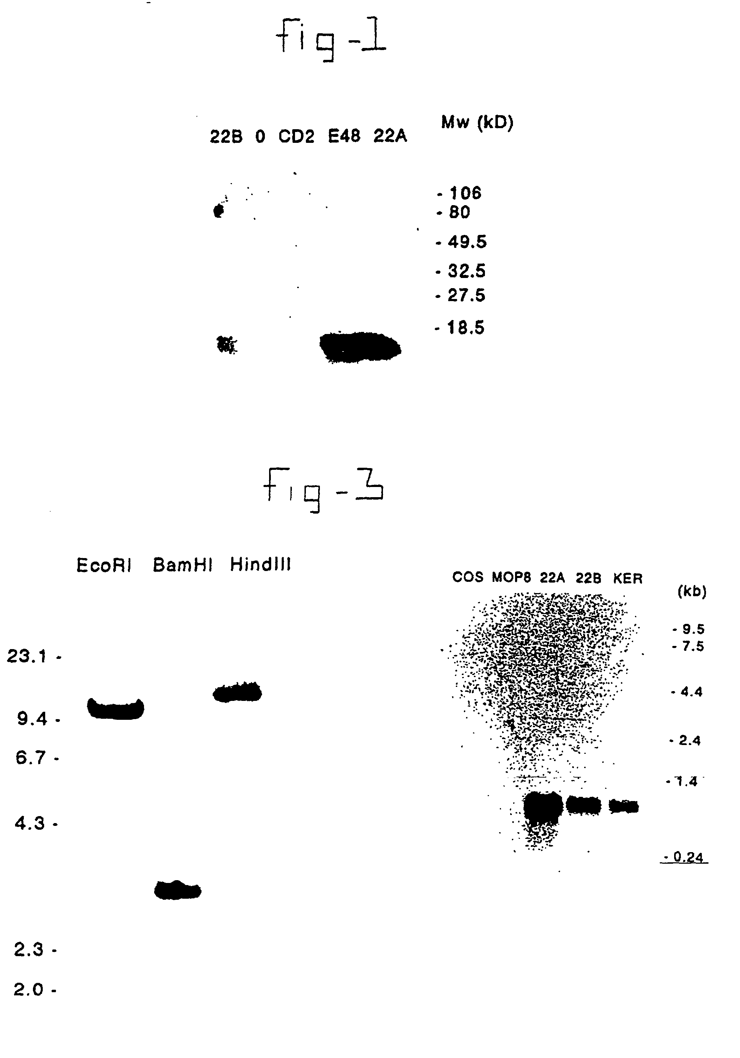

Simultaneously, expression cloning in eukaryotic vector/host systems was set up. CD2 cDNA cloned in the expression shuttle vector pCDM8 (Seed, 1987) and anti-CD2 monoclonal antibody were used as a model system to optimize and simplify the transfection of COS cells, to enhance the detection and subsequent isolation of immunocytochemical stainable cells, and to optimize electrotransformation of *E.coli* MC1061/P3 (Rommens et al., 1991; Brakenhoff et al., 1994b). A cDNA library was generated from RNA of UM-SCC-22A, a HNSCC cell line with a high level of E48 antigen expression, in expression vector pCDM8, and electrotransformed in *E. coli* MC1061/P3. A library with a complexity of 7x10⁵, divided in 9 fractions, was generated. Frozen glycerol cultures were prepared from these fractions and similarly DNA was isolated and transfected into 2x10⁶ COS cells. Immunocytochemical staining *in situ* revealed 80 positive clones. Electrotransformation of Hirt extracted DNA and rescreening of clones was successful for the isolation of the CD2 clone used as a control, but not for the isolation of the E48 cDNA, probably due to COS cell induced damage of the cDNA clones. By screening progressively smaller pools of bacterial colonies one of the positive clones could be enriched to 100% purity. Although this is a laborious method it is completely reliable as the shuttling of plasmid DNA through COS cells is omitted: the transfection and immunocytochemical staining are used only to detect a positive pool of clones. We were able to clone the E48 antigen encoding cDNA in six steps. Transfection of the clone in COS cells and Western blotting of the cell extracts proved that it encoded the correct antigen (Figure 1). The clone was sequenced, the open reading frame was determined and the location of a ribosome binding site and a signal peptide were analyzed by computer calculations. Three putative cleavage sites for a signal peptide were found, of which one would result in a protein with the amino terminal sequence LRCHVCTSSSNCK (Sequence id 2). As indicated earlier, microsequencing of the purified protein revealed the aminoterminal sequence LRC(?)HVVTSSSNNK, indicating that this cleavage site is most likely correct. Not only the 3rd amino acid appeared to be a cysteine but the 6th and the 12th as well. Re-evaluation of the sequencing chromatograms indicated that the signal of the 6th amino acid could indeed be the residual signal of the 5th valine (V), and that the signal of the 12th amino acid could be the residual signal of the 11th asparagine (N).

The E48 cDNA sequence has an open reading frame of 128 amino acids from base 24-408, encoding a core protein with a molecular weight of 13,286 kD. In the amino acid sequence 2 theoretical phosphorylation sites (Thr 20 and Ser 41), and 3 putative myristylation sites (Gly 75. 80 and 114 respectively) are present (Sequence id 2).

The putative poly-adenylation signal GATAAA of the E48 encoding cDNA is not in agreement with the general consensus sequence AATAAA or ATTAAA^{106,107,128}. The activity of poly(A) polymerase on this signal sequence GATAAA has been measured *in vitro* by others, and appeared to be only 10% as compared to the consensus sequence¹³⁰. To exclude that this non-consensus sequence was due to reverse transcriptase infidelity, 5 additional clones were isolated from other fractions of the cDNA library by colony hybridization, and sequenced. The sequences revealed that all clones were of different length, but that in all clones the GATAAA sequence appeared to be the most probable poly-adenylation signal. Furthermore, a clone was isolated from a genomic DNA library in lambda EMBL3⁶⁷ and the BamHI fragments hybridizing with the cDNA were subcloned and sequenced. Sequence analysis of the genomic clone could indeed confirm this putative poly-adenylation sequence. The use of polyadenylation signals not applying to the consensus rules is not specific for this antigen only. Similar irregular sequences have been observed for the CD59 gene as well, although in this case a consensus signal sequence was also found to be present¹²⁴.

Data base searching indicated a high sequence similarity of the human E48 antigen with the mouse ThB antigen, the family of the Ly-6 antigens and human CD59^{99, 76, 92, 102, 82}. The ThB antigen and the family of Ly-6 antigens are all 15-20 kDa membrane bound proteins expressed on lymphocytes, and anchored with a glycosyl-phosphatidylinositol anchor to the membrane. The molecules are thought to be involved in signal transduction, but the corresponding ligands are unknown. The human CD59 molecule is expressed on erythrocytes and is involved in the inhibition of autologous complement attack.

Northern blotting experiments revealed that the E48 gene is exclusively expressed in squamous cell carcinoma cell lines and squamous epithelia (Table I and figure 3 right). The transcript is approximately 0.8 kb in length. Splice variants could not be detected by Northern blotting, not even after overexposure of the autoradiogram. There appears to be a direct relationship between the antigen expression as detected by Western blotting and immunocytachemistry (20% of the UM-SCC-22B cells, and 100% of the UM-SCC-22A cells are positive; see also Figure 1), and the relative amount of E48 mRNA in these cells.

### The E48 antigen is a GPI-anchored membrane protein

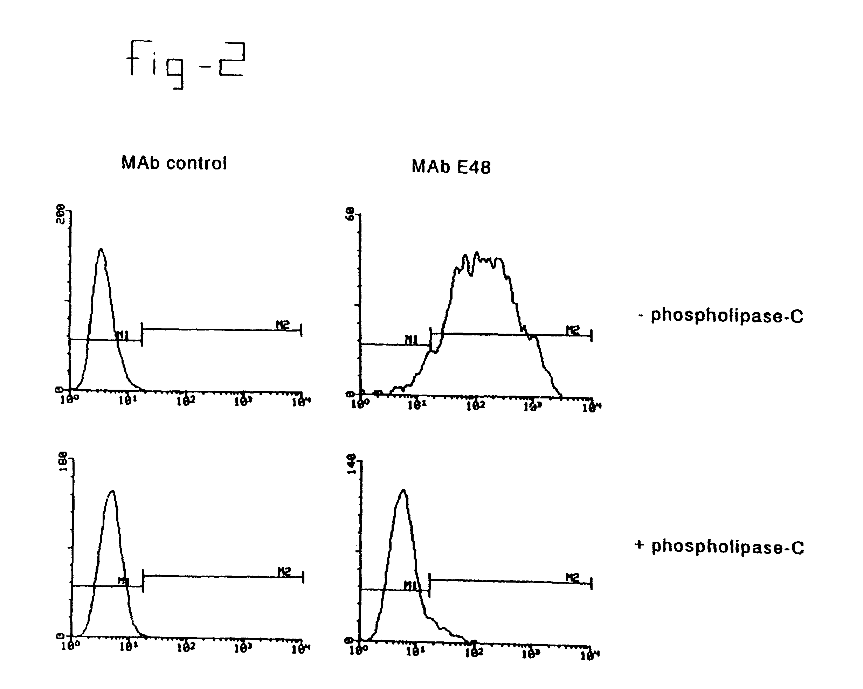

The homology with the Ly-6 family, proteins attached to the membrane with a glycosyl-phosphatidylinositol (GPI)-anchor, suggested that the E48 antigen might be attached similarly. The presence of a GPI-anchor was measured by phosphatidylinositol-(PI)-specific phospholipase-C treatment of antigen positive cells, a method often used to demonstrate the GPI-anchoring of a surface protein⁷⁵. Extracts of PI-specific phospholipase-C treated COS cells transfected with the E48 encoding cDNA were analyzed by Western blotting (not shown), and PI-specific phospholipase-C treated UM-SSC-22A cells were analyzed by immunocytochemistry (not shown) and FACS. The flow cytometry analysis is shown in Figure 2. UM-SCC-22A cells treated with anion free buffer and incubated with E48 antibody showed a high fluorescence, whereas after PI-specific phospholipase-C treatment in anion free buffer and subsequent E48 incubation the UM-SCC-22A cells showed a very low level of fluorescence. The analysis was checked by a similar assay on the GPT-anchored M0v18 antigen (folate binding protein) overexpressed on KB cells^{100, 73} (data not shown). These data clearly indicated that the E48 antigen is released from the cell surface by PI-specific phospholipase-C treatment, demonstrating that the protein is GPI-anchored to the plasma membrane.

### Chromosomal localization of the E48 encoding gene

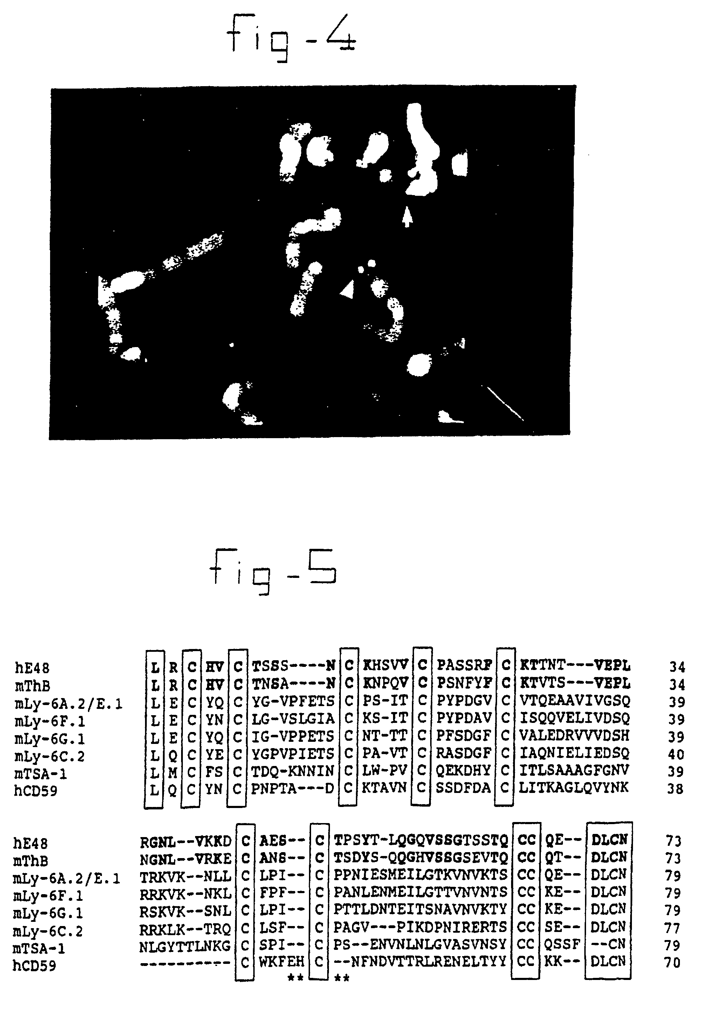

Southern blotting experiments indicated that the E48 antigen is encoded by a single copy gene (Figure 3 left). Subsequent analysis of a panel of 24 somatic cell hybrids revealed significant segregation with human chromosome 8, with a single exception probably due to chromosomal rearrangement after cell fusion (Table II). To obtain a regional localization of the gene on chromosome 8, a clone containing the E48 gene was isolated from a phage library and used for *in situ* hybridization using biotin labeled probes. This analysis clearly showed two regions of hybridization, one on chromosome 8q24-qter and one on chromosome 15q22 (Figure 4). Since the somatic hybrid panel did not show any concordance with chromosome 15, we concluded that the E48 encoding gene is located on chromosome 8, in the 8q24-ter region. The hybridization of the genomic probe (approximately 20 kb in length) to chromosome 15 is most likely due to cross-hybridization with an as yet unidentified pseudogene or with low copy repetitive sequences, as was indicated by Southern blot hybridization (not shown).

### Characterization of the putative mouse homologue, the ThB antigen.

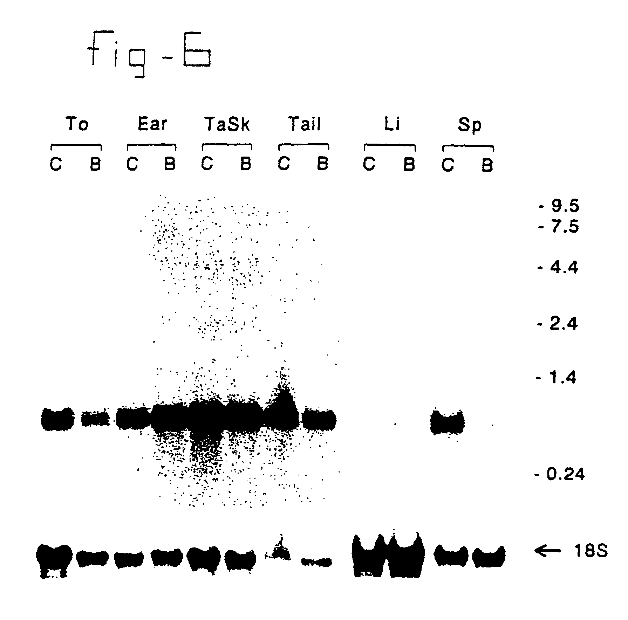

An unexpected observation was the high sequence similarity with the murine ThB antigen, a glycosyl-phosphatidylinositol-anchored molecule expressed on lymphocytes (Figure 5). It had already been described earlier that two allelotypes of the ThB antigen exist: ThB^{h} (C57B1/6 mice) having a high level of expression, and ThB¹ (BALB/c mice) having a low level of expression in lymphocytes⁹². The expression pattern of ThB in squamous tissue, i.e. skin, was not determined. We therefore analysed the ThB expression in various tissues of mice of the ThB^{h} (C57B1/6) and ThB¹ (BALB/c) allelotypes. RNA was isolated, Northern blotted and hybridized with the ThB cDNA as a probe (kindly provided by dr M. Sandrin). In brain, skeletal muscle, colon, heart, kidney and liver no ThB transcripts could be detected, and in spleen, as expected, only a very low level for the ThB¹ (BALB/c) and a relatively high level for the ThB^{h} (C57B1/6) allelotypes could be determined. However, in tongue, tail skin, and ear skin a very high level of ThB RNA could be detected. As shown in Figure 6 the expression of ThB in keratinocytes is not only 10-100 times higher as compared to the spleen of the ThB^{h} allelotype, but is also independent of the mouse strain. Only the expression in the spleen (lymphocytes) is strain dependent, being high in C57BL/6 and low in BALB/c. This expression pattern strongly suggests that the ThB antigen is most likely of critical importance on murine keratinocytes, and not, or less, on lymphocytes. The function of the ThB antigen on murine keratinocytes will most likely be similar to that of the human homologue, the E48 antigen, on human squamous cells. The role of the ThB antigen on lymphocytes remains unclear, although involvement with signal transduction has been suggested⁹². E48 expression on lymphocytes in man has never been observed as yet.

### Functional characterization of the E48 antigen

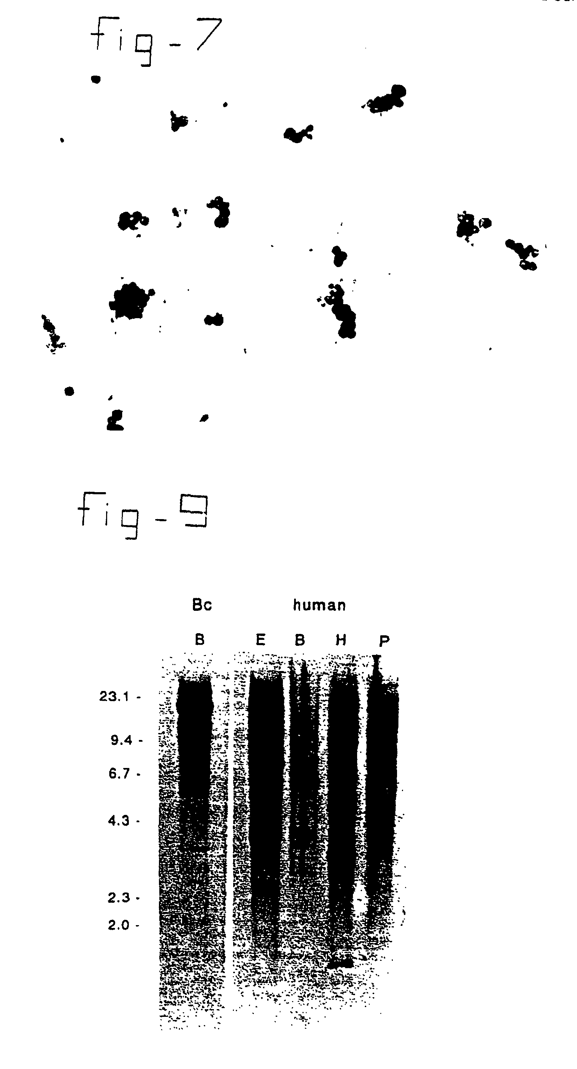

In previous papers the function of the E48 antigen was hypothetically linked to desmosomal cell adhesion. The molecule could be detected in the desmosomal midline by immunoelectronmicroscopy, and, moreover, was shown to influence cell adhesion in *in vitro* model systems¹¹⁴. Although the homology of the E48 sequence with the mouse Ly-6 family would suggest otherwise, the role of the E48 antigen in cell-cell adhesion was clearly demonstrated when the encoding cDNA in expression vector pCDM8 was transiently transfected into MOP-8 cells. SV40-polyoma transformed NIH\3T3 cells. After transfection the cells were cytospun on microscopic glass slides and immunocytochemically stained with the E48 antibody. As shown in Figure 7, all E48 positive cells aggregate, whereas the E48 negative cells remain dispersed, indicating that the molecule is involved in MOP-8 cell-cell interaction. Cells transfected with the WC1 antigen did not show this kind of clustering¹³¹ (not shown).

### DISCUSSION

The E48 antigen is a target molecule for antibody-based immunotherapy, and to envision the results of high dose antibody treatment in patients with squamous cell carcinoma, and to evaluate the potential role of the antigen in carcinogenesis and tumor progression, we decided to clone the cDNA as well as the gene to unravel its function. The cDNA encoding the E48 antigen contained an open reading frame of 401 nucleotides encoding a polypeptide of 13,286 kD. Scanning the E48 polypeptide for post-translational modification sequences revealed two putative protein kinase-C phosphorylation sites and three putative N-myristylation sites. Comparison with a similar analysis on the ThB polypeptide, the putative mouse homologue (see below), revealed that only the N-myristylation site on the glycine at position 80 is conserved. The actual use of these sites should be established by further research.

Surprisingly the E48 antigen appeared to have a very high sequence similarity with the ThB antigen, of which the cDNA was cloned from a C57/B16 mouse spleen cDNA library⁹². This antigen belongs to the larger Ly-6 family of 13-15 kDa surface proteins, GPI-anchored to the membrane and initially identified on murine lymphocytes (See also Figure 5). The E48 antigen is the first member of the human family with perhaps the exception of the CD59. The homology with the Ly-6 family is not only confined to the sequence similarity and the comparable expression pattern (see below), but also to the chromosomal localization of the genes. The E48 gene was assigned to human chromosome 8 in the q24-qter region, where also the myc gene has been mapped. The various members of the mouse Ly-6 family have all been mapped to mouse chromosome 15, in proximity of the myc gene^{154, 92, 102, 82}. These observations stress the homology of the E48 antigen with the mouse ThB antigen, since this part of human chromosome 8 is synthenic with mouse chromosome 15⁷⁸ (Genome Database). It should be noted that the other putative member of the human family of the Ly-6 like GPI-anchored antigens, CD59, is located on chromosome 11p13^{83, 63}. In addition, the sequence similarity of the coding regions of the CD59 and E48 genes is below 50% suggesting that these genes, if they have arisen from a common ancestor, must have been separated long ago in evolution.

It seems obvious that, as in mice, a family of Ly-6 antigens exists in man, most likely clustered at the chromosomal region 8q24-qter. Although several members of the murine Ly-6 gene family were already cloned in 1987. the identification of the human family has been unsuccessful, probably due to similar technical difficulties as we encountered in the molecular cloning of the E48 encoding cDNA. At the time amino acid sequences of the E48 antigen were obtained degenerated oligonucleotides were synthesized for PCR and screening of cDNA libraries based on the sequence LRCHVVT. As the homology with the ThB antigen could already be detected at the protein level (being LRCHVCT) we also generated primers based upon the ThB sequence, as well as the consensus sequence of most Ly-6 members CCXXDLCN, the sequence attached to the GPI-anchor. Although it was confirmed later that the aminoterminal sequence and the GPI-linked consensus sequence CCXXDLCN of the ThB antigen were similar to those of the E48 antigen, RT-PCR with primers based on these amino acid sequences did not result in the amplification of any specific fragment. This negative result is probably due to the (N)₆ nucleotide sequence in the antisense primer.

What is more, cross-hybridization, which is often used to clone cDNAs and genes from other species, is also difficult. The sequence similarity of the E48 cDNA and ThB cDNA clones is 67%, and we have as yet not been able to find conditions for specific cross-hybridization of the full E48 cDNA probe on Southern blots of genomic murine DNA, and of the full ThB cDNA probe on Southern blots of human genomic DNA (data not shown). However, when the sequence between nt 85-304 of sequence id 1 were used specific cross-hybridisation with mouse as well as human genes could be observed (Figure 9). In addition, most Ly-6 genes within one species cross-hybridize^{96, 82}, and the molecular cloning of other members of the putative human family should be facilitated by the identification of the human ThB gene homologue, and its chromosomal localization. From the mouse locus it has been shown that all Ly-6E.1 hybridizing sequences could be found in a single 650 kb fragment⁹⁶. Isolation of YAC clones from a similar region of chromosome 8 using the E48 cDNA as a probe should most likely enable the molecular cloning of other members by subsequent cross-hybridization or even PCR strategies. Although cross-hybridization on genomic blots is difficult, on YAC clones it should be possible.

A number of hereditable diseases have been mapped to chromosome 8q24-qter, such as the Langer-Giedion syndrome, the brachio-otorhinolaryngeal syndrome, the trichorhinolaryngeal syndrome I and the epidermolysis bullosa simplex (OGNA). Some of the clinical features. especially those related to the skin and hair phenotypes might be explained by the deletion of the E48 gene in these genetic diseases⁷⁸ (MIM [Mendelian inheritance of man] database). Particularly when a larger Ly-6 gene family is present at this locus, various hereditable genetic disorders could be due to (partial) deletion of this chromosomal region. Experimental proof of the involvement of the other putative human Ly-6 antigens in these syndromes could only be evaluated in gene targeting experiments in mice.

As indicated by Northern blotting and hybridization, the expression of the E48 antigen is confined to squamous tissues. In mice the putative homologue, ThB. is expressed in lymphocytes as well. although at a much lower level. Moreover, on lymphocytes the expression level is mouse strain dependent, and two phenotypes. ThB^{h} and ThB¹ have been described⁹². We show here that the expression of the ThB antigen appears to be much higher in squamous tissues, and, what is more, independent of the mouse strain. The variation of ThB expression in lymphocytes between the two mouse strains could be explained by a possible difference in a transcription factor recognition sequence in the promoter/enhancer sequence of the two alleles ThB^{h} and ThB¹. The promoter/enhancer sequences of the ThB alleles could therefore bind lymphocyte transcription factors with different affinities, resulting in different levels of ThB expression. A similar conclusion has recently been reported by Gumley et al.⁹¹ to explain the two allelotypes. This hypothesis assumes that the chromosomal domain containing the Ly-6 gene family is accessible for transcription factors in lymphocytes, which can be concluded from the fact that several members of the gene family, such as the Ly-6A.2 gene, are expressed in lymphocytes. In contrast, the promoter sequence of the human gene E48 most likely does not bind any lymphocyte transcription factors, and is therefore not expressed in cells of the lymphocyte lineage.

The ThB/E48 antigen is not the only Ly-6 antigen expressed on keratinocytes. The Ly-6A.2 antigen has also been detected on mouse keratinocytes⁹⁵. and, what is more, other members of the Ly-6 family are expressed in various other tissues and organs^{74, 64, 82, 89, 95}. We have explored the expression of CD59 in human keratinocytes by Northern blotting and immunohistochemistry, but have found only a very low expression level on keratinocytes (data not shown). These and other published data will make it necessary to consider renaming the antigens. hThB: human Thymocyte and B-cell antigen, is not a very logical name for the E48 antigen, if it is exclusively expressed in keratinocytes. We would therefore propose to rename the Ly-6 antigens into CRG proteins: Cysteine-Rich GPI-anchored proteins, and, for the time being, add part of the original name. When the genes encoding these proteins have all been cloned from one species, a definite nomenclature could be agreed, for instance in the direction of trancription, or, if the directions of transcription are different, in the direction from centromere to telomere. A similar consensus has been reached in the nomenclature of the γ-crystallin gene family in various mammalian species⁶¹. In addition we would like to add a prefix to the name to indicate the species from which the antigen has been identified. mCRG-A2/E1 mCRG-C2, mCRG-F2, mCRG-G2 (formerly Ly-6A.2/E.1, Ly-6C.2, Ly-6F.2, Ly-6G.2, respectively; it should be noted that Ly-6A.2 and E.1 are most likely alleles of one gene), mCRG-TSA (formerly mouse TSA antigen) mCRG-ThB (formerly the ThB antigen), hCRG-48 (formerly, the human E48 antigen), hCRG-CD59 (formerly the CD59 antigen, the first human gene identified but on a different locus). Although we realize that changing names is a difficult and annoying process and should only be performed when there is a consensus in the field¹⁰⁴, we think that the trivial name "E48 antigen" should be adapted as soon as possible.

The function of the human E48 (hCRG-E48) antigen and most likely of the mouse ThB antigen in skin, is apparently different from the reported function of the Ly-6 family of antigens in murine lymphocytes. The members of this larger family of CRG-anchored molecules have been shown to be involved in signal transduction. The murine Ly-6A.2 antigen as well as the human CD59 antigen interacts with tyrosine kinases of the src family and the ThB antigen probably interact with a tyrosine phosphatase, which are all involved in protein phosphorylation cascades^{20,} ^{92, 119, 65, 118}. Cross-linking of the Ly-6 molecules by antibodies resulted in lymphocyte activation and proliferation^{101, 93, 155}. To test the hypothesis that cross-linking of the E48 antigen by its corresponding antibody might cause similar effects, we have measured the effect of the antibody binding on the growth behavior of keratinocytes and squamous carcinoma cells in culture by standard assays. Although a control antibody which has a direct influence on cell proliferation showed considerable effects, the E48 antibody did not¹¹⁵ (data not shown). However, it cannot be excluded that only cross-linking of specific epitopes on these molecules causes signal transduction or that these effects can be determined in (murine) lymhocytes only, or that the culture conditions of keratinocytes are not permissive for these effects.

In model assays it has been demonstrated that the E48 antigen is involved in cell-cell adhesion. The role of a GPI-anchored Ly-6 antigen in adhesion processes is novel. GPI-anchored proteins are thought to be involved in potocytosis: rapid transport processes without formation of endosomes, or signal transduction^{71, 62, 132, 133}. GPI-anchored proteins can form microdomains on the membrane localized in caveolae, and characterized by the coat protein caveolin, although the existence of these domain formations is still controversial^{110, 103} Involvement of GPI-anchored proteins with cell-cell adhesion has been described earlier: the human carcinoembryonic antigen (CEA) is a GPI-anchored adhesion molecule⁷⁹. We have shown that the E48 antigen, transfected into MOP-8 fibroblasts induces cell-cell adhesion, or at least specific cell-cell recognition. Model experiments with stable transfected cell lines should elucidate the precise function of the molecule. The reported observation is consistent with the postulated similarity of the E48 antigen with desmoglein III/dg 4, a putative component of the desmosomal complex^{116,114}, which would explain the induced adhesive behavior of the transfected NIH/3T3 cells. It should be noted, however, that desmoglein III/dg 4 (identified in desmosomal preparations of bovine muzzle) had been reported to be glycosylated⁸⁸, whereas the E48 sequence did not reveal any glycosylation sites. These conflicting findings can be explained by the chemical method used by Gorbsky and Steinberg⁸⁸ to detect carbohydrates on the desmosomal components. They described the isolation of desmosomes from bovine muzzle, and the detection of carbohydrates by periodate/dansyl-hydrazine treatment. The reported weak signal of the 22 kD protein could, in view of the data presented here, be explained by the detection of the inositol and the other core sugar residues in the GPI-anchor, all reactive with periodate^{75, 84}. These data demonstrate that the E48 antigen and the 22 kDa desmoglein III/dg 4 indeed may be identical.

It is tempting to speculate that the role of the Ly-6 (CRG) molecules in general is not directly related to the cell proliferation, but only indirectly. It has firmly been established that GPI-anchored molecules are concentrated in specialized membrane domains, and the general function of the small cysteine-rich Ly-6 (CRG) molecules could be the formation of these specialized membrane domains together with caveolin^{81, 110, 112, 85}. The different components of the particular domain could explain the different functions: on T-lymphocytes signal transduction, on MDCK and Caco-2 cells transport, and on keratinocytes cell-cell adhesion. It is obvious that this hypothesis should be proven experimentally, but the observation that CD59 on erythrocytes functions in protection against autologous complement attack, and in T-lymphocytes functions in signal transduction, comparable to the mouse Ly-6A.2 molecules, strongly supports the theory⁹⁷.
Summarized, with the molecular cloning of the cDNA encoding the first member of the human Ly-6 family, a tool for the molecular cloning of cDNAs encoding other members of the human family has been made available, enabling the functional characterization of these CRG-anchored antigens in the various cells and tissues of man.

### MATERIALS AND METHODS

### materials

Restriction enzymes were purchased from Boehringer Mannheim (Boehringer Mannheim B.V., Almere, The Netherlands) or Pharmacia (Pharmacia Biotech Benelux, Roosendaal, The Netherlands). DNA polymerase (Klenow fragment) and T4 DNA ligase were from Boehringer Mannheim. AMV reverse transcriptase was from Promega (Promega, Leiden, The Netherlands) and T4 polynucleotide kinase as well as T7 DNA polymerase used for sequencing were from Pharmacia. The reaction conditions were chosen as recommended by the supplier or according to Sambrook et al.¹¹¹. [³²P]-labeled nucleoside triphosphates (3000 Ci/nmol) were purchased from NEN (NEN Research Products. Boston, MA, USA). The expression vector pCDM8 was obtained from Invitrogen (Sanbio, Uden, The Netherlands), and the CD2 cDNA clone in pCDM8 was kindly provided by dr. B. Seed¹¹⁷. The cDNA clone encoding the ThB antigen was kindly provided by dr. M. Sandrin⁹². Kits for sequence analysis on automated systems were obtained from Applied Biosystems (Applied Biosystems B.V., Maarssen. The Netherlands).

### Cell lines and tissue culture

The HNSCC (Squamous Cell Carcinoma of the Head and Neck) cell lines UM-SCC-22A and UM-SCC-22B, used for Northern blotting and cDNA synthesis (UM-SCC-22A) were kindly provided by dr. T.E. Carey (Ann Arbor, MI, USA). The cell lines OVCAR-3. WiDr-29 and A-431 were obtained from dr. H. Haisma. LNCAP from dr. A.A. Geldof, Tera-1 and MCF-7 from dr. G. Giaccone, DOHH2 from dr. J.W. van Oostveen, NB-1 from dr. A.J. Langeveld, U-715 and Raji from dr. A. Dräger, Hela and SiHa from prof. dr. C.J.L.M. Meijer, KB cells from dr. G. Jansen (all from the Free University, Amsterdam, The Netherlands), and PC112 from dr. P.T. van der Saag (Hubrecht Laboratory. Utrecht, The Netherlands). The tissue origin of the aforementioned human cell lines is indicated in Table I. COS-7 cells were obtained form dr. D.H. Joziasse (Free University. Amsterdam) and MOP-8 cells (SV40-polyoma transformed NIH/3T3 cells) from the ATCC (American Tissue Culture Collection).

Cells were routinely cultured in humidified air/5% CO₂ at 37 °C in Dulbecco's modified Eagle's medium (DMEM, Gibco Life Technologies, Breda, The Netherlands), 5% Fetal Calf Serum (HyClone, Logan, Utah, USA), 2 mM L-glutamine, 1% penicillin/streptomycin (Cellect, ICN Biomedicals B.V., Amsterdam. The Netherlands), 16 mM NaHCO₃ and 15 mM HEPES, pH 7.4, or RPMI 1640 (ICN Biomedicals B.V.), 5% Fetal Calf Serum, 2 mM L-glutamine, 1% penicillin/streptomycin.

Primary keratinocytes were isolated from Uvulo-Palato-Pharyngeal surgical specimens as described for foreskin keratinocytes according to ref.⁷². The cells were cultured in Gibco Keratinocyte Growth Medium (KGM) according to the manufacturers instructions.

As checked by immunocytochemistry of cytospin preparations, 100% of the UM-SCC-22A cells, 20% of the UM-SCC-22B cells and none of the COS-7 as well as MOP-8 cells were shown to express the E48 antigen. irrespective of the culture conditions.

### Antigen isolation

The xenograft line HNX-HN was used as source for the isolation of the MAbE-48 defined antigen. The establishment and maintainance of the xenograft line HNX-HN by serial transplantation was performed as described earlier (Gerretsen et al., 1991). Xenografts were cut into small pieces and snap frozen in liquid nitrogen. The frozen material was pulverized in a micro-dismembrator (Braun AG, Melsungen, Germany) and subsequently resuspended in cold extraction buffer (EXB: 20 mM TRIS.HCl, 150 mM NaCl. 1% (w/v) n-octyl-β-D-glucoside, 1 µg/ml pepstatin, 1 µg/ml leupeptin, 1 µg/ml trypsin inhibitor, 1 mM phenylmethylsulfonylfluoride (PMSF). pH 8.0: 3 ml EXB/mg pulverized tissue). All further steps were performed on ice in the presence of the aforementioned protease inhibitors. The suspension was sonicated 3 times for 10 sec at 10.000 Hz, and centrifuged at 4 °C for 1 hr at 100,000g to obtain a clear supernatant. The proteins were precipitated from the clear supernatant by adding 10 volumes of precooled acetone-methanol (1:1). The mixture was allowed to stand for 4 hr with occasional swirling. The precipitated proteins were collected by centrifugation at 100.000g for 1 hr at 4 °C, the pellet was resuspended in precooled ethylether and the precipitated proteins were collected again by centrifugation at 4 °C for 1 hr at 100.000g. After evaporation of the solvent, the proteins were dissolved in the original volume of EXB. After centrifugation of the solution at 100,000g for 1 hr at 4 °C, the E48 antigen was isolated by immunoabsorbtion. Purified MAbE-48 IgG (kindly provided by Centocor Europe Inc.. Leiden, The Netherlands), was coupled to Affigel-10 (BioRad Laboratories B.V., Veenendaal. The Netherlands) in 0.1 M MES (2-[N-morpholino]ethanesulfonic acid), pH 4.8 for 16 hr at 4 °C. Unbound IgG was removed by washing the material twice with MES for 10 min and twice with EXB. The redissolved protein extract was incubated with the immunoabsorbative for 16 hr at 4 °C under continuous gentle agitation, and washed three times with EXB. The immunoabsorbed material was eluted in 3 x 1 ml 50 mM triethanolamine pH 12.5, 1% n-octyl-β-D-glucoside. The eluted fractions containing antigen were concentrated with Centricon-3 microconcentrators, Mw cut-off 3,000 Da (Grace B.V. Amicon Division, Rotterdam, The Netherlands). Approximately 2 µg protein was run on a SDS-PAGE gel under non-reducing conditions, blotted to Problott™ PVDF (polyvinylidene difluoride, Applied Biosystems). After transfer, the proteins on the membrane were stained with 0.1% Amido Black in 1% acetic acid, 40% methanol. The protein band of 20 kDa was cut out and the amino acid sequence determined by a protein sequencer (Applied Biosystems, model 473A) The isolation was followed by Western blotting of samples taken in every step. Small aliquots of the purified antigen were radioiodinated and analyzed for purity.

### PI-specific phospholipase-C treatment and FACS analysis

5x10⁶ UM-SCC-22A and various control cells were washed with anion-free buffer (225 mM sucrose, 20 mM HEPES, pH adjusted to 7.4 with solid MgO and filtered⁹⁴ and treated with 0.3 U PI-specific phospholipase-C (Boehringer Mannheim) in 200 µl anion-free buffer at 37 °C for 1 hr. Cells were subsequently spun on microscopic glass slides, and stained by immunocytochemistry, or washed with PBS (phosphate buffered saline¹¹¹, and prepared for Western blotting or FACS (fluorescence activated cell sorting) analysis. For FACS analysis the treated cells were incubated with the optimal concentration of MAbs in ice-cold PBS/0.1% BSA for 45 min at 0 °C. After washing in ice-cold PBS/0.1% BSA. the cells were incubated with fluorescein-isothiocyanate (FITC) labeled goat anti-mouse immunoglobulin conjugate (Becton-Dickinson. Etten-Leur, The Netherlands) for 30 min at 0 °C in the dark. After washing the cells were analysed immediately by a FACScan (Becton-Dickinson). The assay was checked by the incubation of KB cells. overexpressing the CRG-anchored folate receptor, and the anti-folate receptor antibody MOv18, and various antibody controls^{100,73}.

### SDS-PAGE and Western blotting.

UM-SCC-22B. UM-SCC-22A cells cultured in monolayer to 80% confluency, and transfected COS-7 cells were washed with PBS, detached by trypsinisation and lysed in Laemmli sample buffer without β-mercaptoethanol⁹⁸. Lysates were boiled, centrifuged. and applied on a miniprotean II system (BioRad) for sodium dodecyl sulphate polyacrylamide gel electrophoresis performed as described by Laemmli⁹⁸. using a 12.5% slab gel. Electrophoretic transfer of proteins from polyacrylamide gel to nitrocellulose (Schleicher and Schüll, Dassel, Germany) or PVDF was performed by a mini-transblot electrophoretic cell (Bio-Rad), at 100V for 1 hr¹¹¹. After transfer, the nitrocellulose membrane was cut into strips and incubated at room temperature with 2% BSA in PBS to block free binding sites on the membrane. The "blocked" nitrocellulose membrane strips were incubated for 1 hr with ¹²⁵I-labeled mMAbE-48 (100.000 cpm/ml). Finally, nitrocellulose strips were washed 3 x 10 win with PBS/0.05% tween-20, dried, and autoradiographed by exposure to Kodak X-AR 5 (Kodak, Odijk. The Netherlands) film for 1 day at -70 °C.

### Probes

DNA fragments were separated from plasmid sequences by "Freeze and Squeeze". In short, DNA digests were run on a NA agarose gel (Pharmacia), the bands were cut out of the gel and frozen. The frozen blocks were put between parafilm and the solution was squeezed out (usually with a glass plate), followed by phenol/chloroform/isoamyl alcohol (25;24:1) extraction and ethanol precipitation¹¹¹. Probes were made by labeling the isolated inserts with [α-³²P]dCTP (NEN research products, 3000 Ci/mmol) through multiprimed elongation⁸⁰.

### DNA isolation, Southern blotting and hybridization

DNA was isolated from tissue culture cells according to Brakenhoff et al.⁶⁶. 10-15 µg of DNA was digested to completion with restriction enzymes, loaded on a 0.7% agarose gel and electrophoresed in TAE buffer according to ref.¹¹¹. The DNA was denatured by soaking the gel in 0.4 M NaOH. 0.6 M NaCl for 30 min, and blotted by capillary transfer to Genescreen-plus filters (NEN research products) in the same solution. After transfer the blot was neutralized in 1 M NH₄ Acetate/0.02 M NaOH for 5 min and washed in 2x SSC. The filter was baked for 2 hr at 80 °C. prehybridized in 7% SDS, 0.5 M sodiumphosphate buffer, 2 mM EDTA, pH 7.0 for 2 hr at 65 °C, and after addition of the denatured probe hybridized at 65 °C for 16 hr. Filters were washed twice with 2x SSC, 0.2% SDS and twice with 0.2x SSC, 0.2% SDS at 65 °C for 15 min, and the bands were visualized by autoradiography with Kodak X-AR 5 film using intensifying screens.

### RNA isolation and Northern blotting

Total RNA was isolated from cultured cells according to Gough (1988), and from tissues using RNAzol (Cinna Biotecx: Campro Scientific, Veenendaal. The Netherlands). 20 µg of total RNA was loaded on a 1% agarose formaldehyde gel and electrophoresed in MOPS buffer essentially as described by Sambrook et al.¹¹¹. The RNA was Northern blotted by capillary transfer in 10xSSC¹¹¹ onto Genescreen plus filters, and hybridized as described above.

### Transfection and imnunocytochemistry

The DEAE-dextran transfection of COS-7 cells occurred essentially according to Brakenhoff et al.⁶⁸. COS-7 cells were trypsinized and plated 1-2 days prior to transfection in a 25 cm² culture flask (Greiner, Alphen a/d Rijn, The Netherlands). The next day cells were trypsinized, counted and 1x10⁶ cells were resuspended in 0.5 ml RSC: RPMI 1640 (ICN Biomedicals B.V.), supplemented with 100 µM chloroquine (Promega) and 2% FCS (Hyclone). 5 to 10 µg DNA (1 µg/µl) was added to 2 ml RSC and mixed with 2 ml RSC-DEAE: 800 µg/ml DEAF dextran (Promega) in RSC. After two min incubation at room temperature the cells resuspended in RSC were added, and the suspension was incubated for two hr in the tissue culture incubator under 5% CO₂ at 37 °C. The cells were subsequently spun at 800g for 5 min and resuspended in 10 ml DMEM. 2x10⁵ cells (2 ml) or 2x10⁴ cells (200 µl) were added to 18 ml or 1.8 ml DMEM and seeded on a 70 cm² or 9.6 cm² dish (Greiner. petridishes TC), respectively. After 48-72 hr the cells were washed with PBS and air-dried.

The transfected cells were fixed for 10 min with ethanol 96%. The dishes were washed twice with PBS (5 min), pre-incubated for 15 min with 2% normal rabbit serum (NRS; Nordic Immunological Laboratories, Tilburg, The Netherlands) in PBS/1% BSA (Fraction V, Sigma), and subsequently incubated for one hour with an appropriate dilution of the specific MAb in PBS/1% BSA (2 ml per 70 cm² dish or 0.3 ml per 9.6 cm² dish, respectively). The dishes were washed 3x with PBS and subsequently incubated for 30 min with rabbit anti-mouse immunoglobulin (RAM; DAKO. Glostrup, Denmark) diluted 1:25 in PBS/1% NRS/1% BSA. The dishes were washed again 3 times with PBS and incubated for 1 hour with alkaline phosphatase anti-alkaline phosphatase (APAAP; DAKO) diluted according to the supplier in PBS/1% BSA. The dishes were washed again 3 times with PBS, rinsed with alkaline phosphatase buffer (APB: 100 mM TRIS.HCl, 100 mM NaCl. 10 mM MgCl₂, pH 9.5) and incubated for 30 min with nitro blue tetrazolium/5-bromo-4-chloro-3-indolyl phosphate substrate (NBT. BCIP; both from Sigma) in APB (5 µl NBT and 1 µl BCIP stock solution per ml APB; stock solutions: 20 mg/ml NBT in 70% dimethylformamide/30% demineralized water, and 50 mg/ml BCIP in dimethylformamide). Dishes were rinsed with demineralized water and the nuclei stained with nuclear fast red (Sigma) for 10 min (1 g nuclear fast red, 70 gram Al₂(SO₄)₃ per liter demineralized water, boiled in a waterbath and filtered before use). The dishes were rinsed again with demineralized water and evaluated under a microscope (Olympus, Paes Nederland B.V., Zoeterwoude, The Netherlands) at a magnification of 50x and 125x. The staining is preserved for at least one week without any loss of intensity.

MOP-8 cells. SV40-polyoma transformed mouse NIH/3T3 cells, were transfected with DEAE dextran essentially as described for COS-7 cells. The amount of DEAE dextran, however, was reduced to 500 µg/ml, the amount of DNA was decreased to 1 µg and the transfection time was reduced to 50 min.

### Construction of an UM-SCC-22A cDNA library in pCDM8

Total RNA was isolated from UM-SCC-22A cells according to Gough⁹⁰. 20 µg was primed with oligo-dT, and used for cDNA synthesis (Promega cDNA kit). The cDNA was ligated to non-complementary EcoRI/BstXI adaptors (Invitrogen; Sanbio) and separated from non-ligated adaptors on Sepharose CL 4B (Pharmacia) spin columns. The fractions containing cDNA larger than 500 bp were used for ligation into pCDM8 vector digested with BstXI, and transformed to E. *coli* MC1061/P3 by electroporation as described by Rommens et al.¹⁰⁹. The library, 7x10⁵ clones, was divided into 9 fractions, grown overnight, glycerol stocks were prepared and DNA was isolated via Qiagen tip-500 columns (Qiagen; Westburg, Leusden, The Netherlands) according to the supplier.

### Isolation of clones by screening progressively smaller pools of bacterial colonies

The 9 fractions of the library were transfected to COS-7 cells, and the cells were immunocytochemically stained with E48 monoclonal antibody as described. The fraction with the smallest number of bacterial colonies and the largest number of positively stained cells was determined, the glycerol stock of that particular fraction was titered and the fraction was divided into ten subfractions of 1/5 of the number of colonies from the parental fraction (thus screening a twofold excess of bacterial colonies). A sample of the inoculated culture was spread on agar plates to determine the exact number of colonies added. The subtractions were grown overnight, frozen glycerol cultures were prepared and DNA was isolated from the same culture, COS cells were transfected and immunocytochemically stained. Repeated cycles of this analysis were performed until the cDNA clone of interest was enriched to homogeneity. DNA isolated by alkaline lysis (Sambrook et al.. 1989) or Qiaprep-spin spin columns (Qiagen; Westburg) are all suited for transfection. A small amount (5%) of CD2 cDNA in pCDM8 was added to all DNA preparations to control the transfection efficiency of each particular sample by staining 10% of the transfected cells on a small dish with anti-CD2 antibody (Sanbio). The E48 antigen encoding cDNA could be isolated in 6 steps.

### Chromosomal localization

Chromosomal assignment of the E48 antigen gene was performed using a panel of well defined human-rodent somatic cell hybrids using the cDNA as a probe. Isolation of hybrid cell lines and determination of chromosome content has been described previously⁶⁶. Regional mapping was performed using fluorescence *in situ* hybridization essentially as described by Suijkerbuijk et al.¹²² with a lambda genomic clone as a probe, and analyzed on a Zeiss Axiophot epifluorescence microscope.

**Table I:**

| **Expression of the E48 gene in tumor cell lines** | | |
|---|---|---|
| In the first column the codes of the human cell lines and tissues are given, in the second the type of tumor and the tissue of origin and in the third the presence of E48 RNA as determined by Northern blotting. | | |

| cell line | | origin positive |
|---|---|---|
| UM-SCC-22A | squamous cell carcinoma | ++ |
| UM-SCC-22B | squamous cell carcinoma | + |
| OVCAR-3 | adenocarcinoma ovary | - |
| WiDr 29 | adenocarcinoma colon | - |
| A431 | epidermoid carcinoma vulva | + |
| LNCAP | adenocarcinoma prostate | - |
| Tera-1 | teratocarcinoma | - |
| MCF-7 | adenocarcinoma breast | - |
| DOHH2 | B-cell lymphoma | - |
| NB-1 | neuroblastoma | - |
| U715 | B-cell lymphoma | - |
| Raji | Burkitt lymphoma | - |
| Hela | epitheloid carcinoma cervix | - |
| SiHa | squamous carcinoma cervix | + |
| PC112 | pheochromocytoma | - |

| primary cells/tissues | | |
|---|---|---|
| keratinocytes | oral cavity | + |
| peripheral blood | mononuclear cells | - |
| adrenal gland | | - |

**Table II:**

| **Segregation of human chromosomes and/or chromosome specific markers and the E48 cDNA in rodent/human somatic cell hybrids** | | | | | |
|---|---|---|---|---|---|
| Southern blots with DNA isolated from human/rodent cell hybrids were hybridized with the E48 cDNA as probe, and the categories of discordants counted separately. | | | | | |

| chromosome | E48/chromosome; no. of clones | | | | discordancy |
|---|---|---|---|---|---|
| | +/+ | +/- | -/+ | -/- | (%) |
| 1 | 6 | 5 | 5 | 8 | 42 |
| 2 | 4 | 7 | 1 | 12 | 33 |
| 3 | 5 | 6 | 3 | 10 | 38 |
| 4 | 9 | 2 | 3 | 10 | 21 |
| 5 | 6 | 5 | 5 | 8 | 42 |
| 6 | 7 | 4 | 2 | 11 | 25 |
| 7 | 7 | 4 | 6 | 7 | 42 |
| 8 | 11 | 0 | 1 | 12 | 4 |
| 9 | 5 | 6 | 3 | 10 | 38 |
| 10 | 8 | 3 | 4 | 9 | 29 |
| 11 | 5 | 6 | 6 | 7 | 50 |
| 12 | 8 | 3 | 5 | 8 | 33 |
| 13 | 8 | 3 | 3 | 10 | 25 |
| 14 | 7 | 4 | 5 | 8 | 38 |
| 15 | 4 | 7 | 8 | 5 | 63 |
| 16 | 10 | 1 | 6 | 7 | 29 |
| 17 | 10 | 1 | 9 | 4 | 42 |
| 18 | 7 | 4 | 6 | 7 | 42 |
| 19 | 10 | 1 | 5 | 8 | 25 |
| 20 | 9 | 2 | 3 | 10 | 21 |
| 21 | 6 | 5 | 5 | 8 | 42 |
| 22 | 8 | 3 | 6 | 7 | 38 |
| X | 9 | 2 | 4 | 9 | 25 |

### LEGENDS

### Figure 1: Western blot of COS cells transfected with the E48 cDNA clone

COS cells were transiently transfected with no DNA (0), the CD2 cDNA clone in pCDM8 (CD2), and the E48 cDNA clone in pCDM8 (E48), and harvested 48 hr post transfection. Cell extracts of the transfected COS cells, and cell extracts of UM-SCC-22A (22A) and UM-SCC-22B (22B) as positive control, were run on a 12.5% SDS-PAGE gel and electroblotted to nitrocellulose. The blot was incubated with ¹²⁵I-labeled E48 antibody, and exposed to Kodak X-AR 5 film for 1 week using intensifying screens. The pattern of a molecular weight marker in kDa (BioRad) is indicated on the right.

### Figure 2: Flow cytometry analysis of E48 antigen expression on UM-SCC-22A cells with and without PI-specific phospholipase-C treatment

UM-SCC-22A tumor cells were trypsinized and incubated in anion free buffer with (+) or without (-) PI-specific phospholipase-C, followed by incubation with MAbE-48 or an isotype matched control antibody (MAb control), and goat anti-mouse FITC immunoglobulin conjugate. The cells were analyzed by flow cytometry, and the corresponding histograms are shown. On the X-axis, arbitrary units of fluorescence are indicated, and on the y-axis the number of cells counted. The M1/M2 gates were adjusted to 0.5% positive cells with the negative control antibody. Note that the E48 antigen is released from the plasma membrane of the UM-SCC-22A cells by PI-specific phospholipase-C treatment.

### Figure 3: Southern blot and Northern blot hybridized with the E48 cDNA

left) 10 µg human genomic DNA was digested with EcoRI, BamHI and HindIII respectively, run on a 0,7% agarose gel. Southern blotted and hybridized with E48 cDNA as a probe under moderate stringent conditions. After hybridization the blot was exposed to Kodak X-AR 5 film for 64 hr using intensifying screens. The fragment lengths in kilobases of lambda DNA cut with HindIII are indicated on the left.

right) 20 µg total RNA isolated from COS-7. MOP-8. UM-SCC-22A, UM-SCC-22B cells and cultured primary keratinocytes were run on an 1% agarose/formaldehyde gel. Northern blotted and hybridized with the E48 cDNA as a probe. The blot was exposed to Kodak X-AR 5 film for 24 hr using intensifying screens. The length in kilobases of an RNA standard (BRL, Gibco Life Technologies) is indicated on the right.

### Figure 4: Regional localization of the E48 gene on human chromosome 8q24-qter

Metaphase spreads of human chromosomes were hybridized *in situ* with a biotin-labeled EMBL3 lambda clone containing the E48 gene. The chromosomes were counterstained and analyzed as described by Suijkerbuijk et al.¹²². Note the white spots indicated with an arrow in the centromeric region of chromosome 15, and indicated with an arrowhead in the telomeric region (8q24-qter) of chromosome 8.

### Figure 5: Homology of the human E48 antigen with the murine Ly-6 antigen family and the human CD59 antigen

Sequence similarity of the murine Ly-6 family and the human CD59 protein with the human E48 antigen as determined by the computer program Clustal, PCgene. Intelligenetics. Conserved residues are boxed, only the sequences of the proteins as expressed on the cell membrane are shown. The gaps indicated with asterisks were additionally introduced to align the 7th conserved cysteine residue. Identical residues of the mouse ThB and the human E48 antigen are indicated in bold. Sequences were taken from LeClair et al.⁹⁹, Palfree et al.¹⁰⁴, Davies et ₐl.⁷⁶, Gumley et al.⁹², Fleming et al.⁸², MacNeil et al.¹⁰².

### Figure 6: Expression of the ThB antigen in squamous epithelia of the mouse

RNA was isolated from C57B1/6 (C) and BALB/c (B) tongue (To), ear, epidermis of tail skin (TaSk), tail skin (Tail), liver (Li) and spleen (Sp). Approximately 10 µg (Tail 3 µg, Ear 7 µg) was run on a 1% agarose/formaldehyde gel, blotted to genescreen plus and hybridized with the ThB cDNA as probe under moderate stringent conditions. The blot was exposed for 64 hr (To, Ear. Task, Tail) or 2 weeks (Li. Sp) to Kodak X-AR 5 film using intensifying screens. Note the large difference in the level of ThB transcripts in spleen RNA (Sp) between the ThB^{h} (C57BL/6, lane C) and ThB¹ (BALB/c. lane B) mouse strains, whereas the differences in RNA level between these strains in squamous tissues (To, Ear, TaSk, Tail) is negligible. The blot was subsequently hybridized with an 18S rRNA probe, and exposed for 2 hr to Kodak X-AR 5 film. The hybridizing bands are indicated at the lower panel.

### Figure 7: Functional characterization of the E48 antigen

MOP-8 cells were transiently transfected with the E48 cDNA in pCDM8. They were removed from the culture flask after 48 hr by mild trypsinization, and spun on microscopical glass slides, coated with 0.1% poly-L-lysine, and immunocytochemically stained by MAbE-48 and APAAP. Note the striking clustering of transfected and E48 expressing cells, while the non-transfected cells are dispersed.

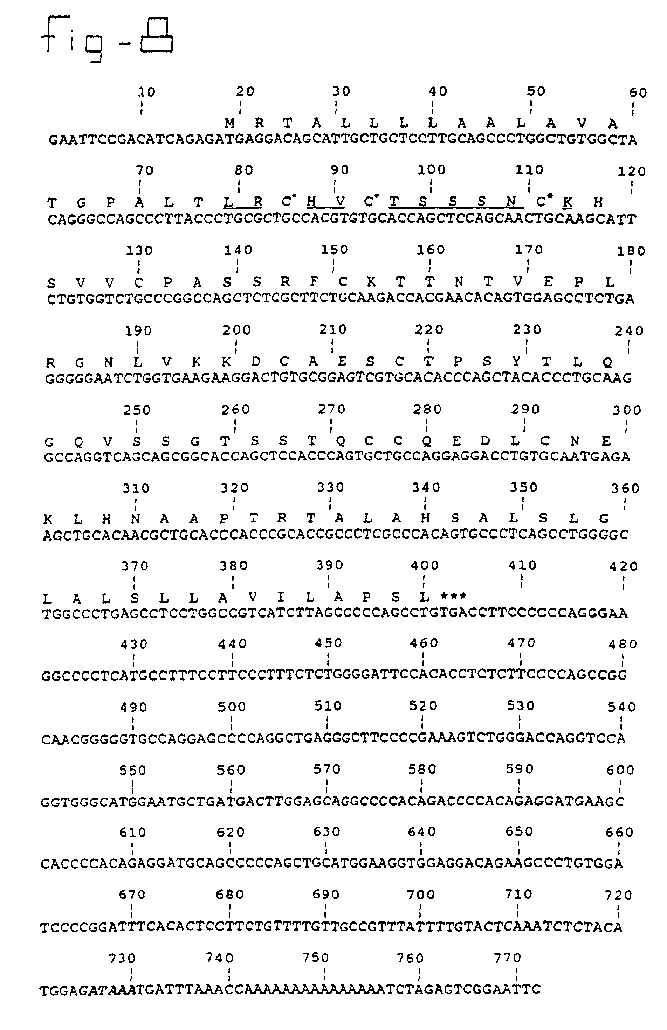

### Figure 8: Sequence of the E48 encoding cDNA and amino acid sequence

The sequence of the E48 encoding cDNA clone, isolated by expression cloning in COS cells, is given in capitals. The underlined amino acid sequences were determined by amino acid sequencing of the purified antigen except for the cysteines indicated width C*. The putative polyadenylation sequence is indicated in bold and italicized. The stopcodon is indicated with asterisks. The XbaI site at the 3' end as well as the EcoRI sites at the 5' and 3' ends of the cDNA are derived from the oligo(dT) primer-adaptor and BstXI/EcoRI adaptors used for the construction of the cDNA library, respectively.

### Figure 9: Cross-hybridisation of murine and human genomic DNA on Southern blots with the E48 probe (nt 85-304 sequence id 1)

Genomic DNA isolated from the liver of a Balb1C (Bc) mouse or human blood (human) were cut with BamHI (B), EcoRI (E), HindIII (H) or Pst I (P), run on an agarose gel, Southern blotted, hybridized and autoradiographed.

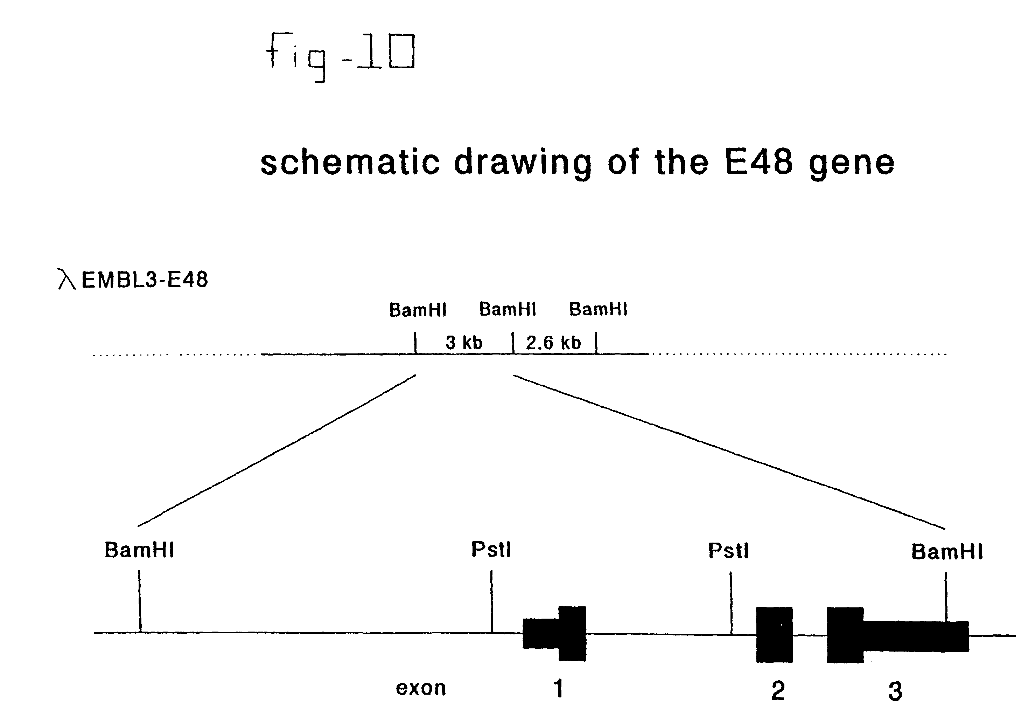

### Figure 10: Schematic Drawing of the E48 gene

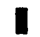 non coding exon sequences
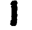 coding exon
― intron/gene flanking sequences

### REFERENCES

- 1.: Muir C, Weiland L. Upper aerodigestive tract cancers. Cancer 1995;75:147-53.
- 2.: Boring CC, Squires TS, Tong T, Montgomery, S. Cancer statistics 1994. CA Cancer J Clin 1994;44:7-26.
- 3.: Blitzer PH. Epidemiology of head and neck cancer. Semin Oncol 1988;15:2-9.
- 4.: Parkin DM, Laara E, Muir CS. Estimates of worldwide frequency of sixteen major cancers in 1980. Int J Cancer 1088;41:184-97
- 5.: Coordinating council of comprehensive cancer in the Netherlands 1991. Third report of the Netherlands Cancer Registry. Utrecht: LOK, 1994.
- 6.: Verham GA, Crowther JA. Head and neck carcinoma - stage at presentation. Clin Otolaryngol 1994;19:120-4.
- 7.: Carter RL, Tanner NSB, Clifford P, Shaw HJ. Perineural spread in squamous cell carcinomas of the head and neck; a clinicopthological study. Clin Otolaryngol 1979;4:271-81.
- 8.: Poleksic S, Kalwaic HJ. Prognostic value of vascular invasion in squamous cell carcinoma of the head and neck. Plast Reconstr Surg 1978;61:234-40.
- 9.: Carr I. Lymphatic metastases. Cancer Metastasis Rev 1983;2:307-17.
- 10.: Jones AS. Prognosis in mouth cancer: tumour factors. Oral Oncol. Eur J Cancer 1994;30B;8-15.
- 11.: Regueiro CA. Aragon G, Millan I. Valcarcel FJ, De la Torre A, Magallon R. Prognostic factors for local control, regional control and survival in oropharyngeal squamous cell carcinoma. Eur J Cancer 1994;30A:2060-7.
- 12.: Gamel JW, Jones AS. Squamous carcinoma of the head and neck: cured fraction and median survival time as function of age, sex, histologic type and node status. Br J Cancer 1993;67:1071-5.
- 13.: Snow GB. The NO neck in head and neck cancer patients. Eur Arch Otorhinolaryngol 1993;250:423.
- 14.: Leemans CR, Tiwari R, Nauta JJP, van der Waal I, Snow GB. Regional lymph node involvement and its significance in the development of distant metastases in head and neck carcinoma. Cancer 1993;71:452-6.
- 15.: Cerezo L, Millan I, Torre A, Aragon G, Otero J. Prognostic factors for survival and tumor control in cervical lymph node metastases from head and neck cancer. A multivariate study of 492 cases. Cancer 1992;69;1224-34.
- 16.: O'Brien CJ, Smith JW, Soon S-J, Urist MM, Maddox WA. Neck dissection with and without radiotherapy: prognostic factors, patterns of recurrence, and survival. Am J Surg 1986;152;456-63.
- 17.: Snow GB, Annyas AA, Slooten EA van, Bartelink H, Hart AAM. Prognostic factors of neck node metastasis. Clin Otolaryngol 1982;7:185-92.
- 18.: Richard JM, Sancho-Garnier H, Micheau C, Saravane D, Cachin Y. Prognostic factors in cervical lymph node metastasis in upper respiratory and digestive tract carcinomas: a study of 1,713 cases during a 15-year period. Laryngoscope 1987;97;97-101.
- 19.: Stell PM. Prognosis in laryngeal carcinoma: tumour factors. Clin Otolaryngol 1990;15:69-31.
- 20.: Mantravadi RVP, Skolnik EM, Haas RE, Applebaum EL. Patterns of cancer recurrence in the postoperatively irradiated neck. Arch Otolaryngol 1983;109:753-6.
- 21.: Tannock IF, Browman G. Lack of evidence for a role of chemotherapy in the routine management of locally advanced head and neck cancer. J Clin Oncol 1986;4;1121-6.
- 22.: Som PM. Lymph nodes of the neck. Radiology 1987;165:593-600.
- 23.: Johnson JT, Barnes EL, Myers EN, Schramm VL, Borochovitz D, Sigler BA. The extracapsular spread of tumors in cervical node metastasis. Arch Otolaryngol 1981;107:725-9.
- 24.: Carter RL, Bliss JM, Soo K-E, O'Brien CJ. Radical neck dissections for squamous cell carcinomas: pathological findings and their clinical implications with particular reference to transcapsular spread. Int J Radiat Oncol Biol Phys 1987;13:825-32.
- 25.: Spiro RH. Alfonso AE, Farr HW, Strong EW. Cervical node metastases from epidermoid carcinoma of the oral cavity and oropharynx. A critical assessment of current staging. Am J Surg 1974;128:562-7.
- 26.: Grandi C, Alloisio M, Moglia D, Podrecca S. Sala L, Salvatori P, Molinari R. Prognostic significance of lymphatic spread in head and neck carcinomas: therapeutic implications. Head Neck
- 27.: Surg 1985;6:67-73. Jones AS, Roland NJ, Field JK, Phillips DE. The level of cervical lymph node metastases: their prognostic relevance and relationship with head and neck squamous carcinoma primary sites. Clin Otolaryngol 1994;19:63-69.
- 28.: Merino OR, Lindberg RD, Fletcher GH. An analysis of distant metastases from squamous cell carcinoma of the upper respiratory and digestive tracts. Cancer 1977;40;145-51.
- 29.: Berger DS, Fletcher GH. Distant metastases following local control of squamous cell carcinoma of the nasopharynx, tonsillar fossa and base of tongue. Radiology 1971;100:141-3.
- 30.: Vikram B, Strong EW, Shah JP, Spiro R. Failure at distant sites following multimodality treatment for advanced head and neck cancer. Head Neck Surg 1984;6:730-3.
- 31.: Dennington ML, Carter DR. Meyers AD. Distant metastases in head and neck carcinoma. Laryngoscope 1980;90:196-201.
- 32.: Nishijima W, Takooda S. Tokita N, Takayama S, Sakura M. Analyses of distant metastases in squamous cell carcinoma of the head and neck and lesions above the clavicle at autopsy. Arch Otolaryngol Head Neck Surg 1993;119:65-68.
- 33.: Zbären P, Lehmann W. Frequency and site of distant metastases in head and neck squamous cell carcinoma. An analysis of 101 cases at autopsy. Arch Otolaryngol Head Neck Surg 1987;113:762-4.
- 34.: Head and Neck Contracts Program. Adjuvant chemotherapy in advanced head and neck squamous carcinoma. Final report of the Head and Neck Contracts Program. Cancer 1987;60:301-11.
- 35.: Ellis ER. Mendenhall WM, Rao PV, Parsons JT, Sprangler AE, Million RR. Does node location affects the incidence of distant metastases in head and neck squamous cell carcinoma? Int J Radiat Oncol Biol Phys 1989;17:293-97.
- 36.: Snow JB. Surgical management of head and neck cancer. Semin Oncol 1988;15:20-8.
- 37.: Haughey BH, Gates GA, Arfken CL, Harvey J. Meta-analysis of second malignant tumors in head and neck cancer: the case for an endoscopic screening protocol. Ann Otol Rhinol Laryngol 1992;101:10512.
- 38.: Madison MT. Remley KB. Latchaw RE, Mitchell SL. Radiologic diagnosis and staging of head and neck squamous cell carcinoma. Radiol Clin North Am 1994;32:163-81.
- 39.: Muraki AS, Mancuso AA, Harsberger HR, Johnson LP, Meads GB. CT of the oropharynx, tongue base, and floor of mouth: normal anatomy and range of variations, and applications in staging carcinoma. Radiology 1983;148:725-31.
- 40.: Watkinson JC, Johnston D, Jones N, Coady M, Laws D. Allen S, Hibbert J. The reliability of palpation in the assessment of tumours. Clin Otolaryngol 1990;15:405-9.
- 41.: Sako K, Pradier RN, Marchetta FC, Pickren JW. Fallibility of palpation in the diagnosis of metastases to cervical nodes. Surg Gyn Obst 1964;113:989-90.
- 42.: Ali S, Tiwari RM, Snow GB. False positive and false negative neck nodes. Head Neck Surg 1985;8:78-82.
- 43.: Brekel van den MWM. Castelijns JA, Croll GA. Stel HV, Valk J. Waal van der I, Golding RP. Meijer CJLM, Snow GB. Magnetic resonance imaging vs palpation of cervical lymph node metastasis. Arch Otolaryngol Head Neck Surg 1991;117:66-73.
- 44.: Brekel van den MWM, Castelijns JA, Stel HV, Golding RP, Meijer CJLM. Snow GB. Lymph node staging in patients with clinically negative neck examinations by ultrasound and ultrasound-guided aspiration cytology. Am J Surg 1991;162:362-6.
- 45.: Castelijns JA. Brekel van den MWM. Magnetic resonance imaging evaluation of extracranial head and neck tumors. Magn Res Q 1993;9:113-28.
- 46.: Brekel van den MWM. Castelijns JA. Stel HV, Golding RP, Meijer CJLM. Snow GB. Modern imaging techniques and ultrasound guided aspiration cytology for the assessment of the neck node metastases; a prospective comparitive study. Eur Arch Otorhinolaryngol 1993;250:11-7.
- 47.: Baatenburg de Jong RJ, Rongen RJ, Lameris JS. Metastatic neck disease. Palpation vs ultrasound examination. Arch Otolaryngol Head Neck Surg 1989;115:689-90.
- 48.: Baatenburg de Jong RJ, Rongen RJ, Verwoerd CDA, Overhagen van H, Lameris JS, Knegt P. Ultrasound-guided fine needle aspiration biopsy of neck nodes. Arch Otolaryngol Head Neck Surg 1991;117:402-4.
- 49.: Friedmann M, Roberts N, Kirschenbaum GL, Colombo J. Nodal size of metastatic squamous cell carcinoma of the neck. Laryngoscope 1993;103:854-6.
- 50.: Stern WBR, Silver CE, Zeifer BA, Persky MS, Heller KS. Computed tomography of the clinical negative neck. Head Neck 1990;12;109-13.
- 51.: Lenz M, Kersting-Sommerhoff B. Gross M. Diagnosis and treatment of the NO neck in carcinomas of the upper aerodigestive tract: current status of diagnostic procedures. Eur Arch Otorhinolaryngol 1993;250:432-8.
- 52.: Vokes EE, Weichselbaum RR, Lippman SM. Hong WK. Head and neck cancer. New Engl J Med 1993;328:184-94.
- 53.: Ervin TJ, Clark JR, Weichselbaum RR. Multidisciplinary treatment of advanced squamous carcinoma of the head and neck. Semin Oncol 1985;4(suppl 6):71-8.
- 54.: Boysen M, Lovdal 0, Natvig K, Tausjo J, Jacobsen A-B, Evensen JF. Combined radiotherapy and surgery in the treatment of neck node metastases from squamous cell carcinoma of the head and neck. Acta Oncol 1992;31:455-60.
- 55.: Vokes EE. Head and neck cancer. In: Perry MC. ed. The chemotherapy source book. Williams and Wilkins, Baltimore 1992:918-31.
- 56.: Vikram B. Changing patterns of failure in advanced head and neck cancer. Arch Otolaryngol 1984;110:564-5.
- 57.: Goepfert H. Are we making any progress? Arch Otolaryngol 1984;110:562-3.
- 58.: Stell PM, Rawson NSB. Adjuvant chemotherapy in head and neck cancer. Br J Cancer 1990;61:779-87.
- 59.: Taylor SG, Applebaum E, Showel JL, Norusis M, Holinger LD, Hutchinson JC, Murthy AK. Caldarelli DD. A randomized trial of adjuvant chemotherapy in head and neck cancer. J Clin Oncol 1985;3:672-9.
- 60.: Tannock IF, Browman G. Lack of evidence for a role of chemotherapy in the routine management of locally advanced head and neck cancer. J Clin Oncol 1986;4:1121-6.
- 61.: Aarts, H. J. M.. J. T. den Dunnen, J. Leunissen, N. H. Lubsen, and J. G. G. Schoenmakers. 1988. The γ-crystallin gene families: sequence and evolutionary patterns. *J. Mol. Evol.* 27:163-172.
- 62.: Anderson, R. G. W. 1993. Caveolae, where incoming and outgowing messengers meet. *Proc. Natl. Acad. Sci. U.S.A.* 90:10909-10913.
- 63.: Bickmore, W. A., D. Longbottom, K. Oghene, J. M. Fletcher, and V. van Heyningen. 1993. Co-localization of the human CD59 gene to 11p13 with the MIC11 cell surface antigen. *Genomics* 17:129-135.
- 64.: Blake, P. G., J. Madrenas, and P. F. Halloran. 1993. Ly-6 in kidney is widely expressed on tubular epithelium and vascular endothelium and is up-regulated by interferon-gamma. *J. Am. Soc. Nephrol.* 4:1140-1150.
- 65.: Bohuslav, J., T. Cinek, and V. Horejsi. 1993. Large, detergentresistent complexes containing murine antigens Thy-1 and Ly-6 and protein tyrosine kinase p561ck. *Eur. J. Immunol*. 23:825-828.
- 66.: Brakenhoff, R. H., A. H. M. Geurts van Kessel, M. Oldenburg, J. Th. Wijnen. H. Bloemendal, P. Meera Khan, and J. G. G. Schoenmakers. 1990. Human αB-crystallin (*CRYA2*) gene mapped to chromosome 11q12-q23. *Hum. Genet.* 85:237-240.
- 67.: Brakenhoff, R. H., H. M. Hensen, J. G. G. Schoenmakers, and N. H. Lubsen. 1994a. Activation of the γ-crystallin pseudogene in the human hereditary coppock-like cataract. *Hum. Molec. Genet.* 3:279-283.
- 68.: Brakenhoff, R. H., P. M. C. Knippels, and G. A. M. S. van Dongen. 1994b. Optimization and simplification of expression cloning in eukaryotic vector/host systems. *Anal. Biochem.* 218:460-463.
- 69.: de Bree, R., J. C. Roos, J. J. Quak, W. den Hollander, M. W. M. van den Brekel, J. E. van der Wal. H. Tobi, G. B. Snow, and G. A. M. S. van Dongen. 1994a. Clinical imaging of head and neck cancer with ^{99m}Tc-labeled monoclonal antibody E48 IgG or P(ab')₂. *J. Nucl. Med.* 35:775-783.
- 70.: de Bree. R.. J. C. Roos, J. J. Quak, W. den Hollander, A. J. Wilhelm, A. van Lingen, G. B. Snow, and G. A. M. S. van Dongen. 1994b. Biodistribution and pharmacokinetics of radiolabeled monoclonal antibodies E48 IgG and F(ab')₂ in patients with head and neck cancer. *Clin. Cancer Res.* in press.
- 71.: Brown. D. A., and J. K. Rose. 1992. Sorting of CRG-anchored proteins to glycolipid-enriched membrane subdomains during transport to the apical cell surface. *Cell* 68:533-544.
- 72.: Bruynzeel, I., L. M. van der Raaij. D. M. Boorsma, P. De Haan. R. J. Scheper, G. Kraal. and R. Willemze. 1990. Spectrophotometric quantification of cell-cell adherence by an enzyme-linked immuno-cell adhesion assay. *J. Immunol. Meth.* 132:51-56.
- 73.: Coney, L. R., A. Tomassetti, L. Carayannopoulos, V. Frasca, B. Kamen, M. Colnaghi, and V. R. Zurawski. 1991. Cloning of a tumor-associated antigen: MOv18 and MOv19 antibodies recognize a folate binding protein. *Cancer Res.* 51:6125-6132.
- 74.: Cray, C., R. W. Keane, T. R. Malek, and R. B. Levy. 1990. Regulation and selective expression of the Ly-6A/E, a lymphocyte activation molecule, in the central nervous system. *Brain Res. Mol. Brain Res.* 8:9-15.
- 75.: Cross, G. A. M. 1990. Glycolipid anchoring of plasma membrane proteins. *Ann. Rev. Cell*. *Biol.* 6:1-39.
- 76.: Davies A, D. L. Simmonds, G. Hale, R. A. Harrison, H. Tighe, P. J. Lachmann, and H. Waldmann. 1989. CD59, a Ly-6 like protein expressed in human lymphoid cells, regulates the action of the complement membrane attack complex on homologous cells. *J. Exp. Med.* 170:637-654.
- 77.: van Dongen, G. A. M. S. R. H. Brakenhoff, R. de Bree, M. Gerretsen, J. J. Quak, and G. B. Snow. 1994. Progress in radioimmunotherapy of head and neck cancer. *Oncol. Rep.* 1:259-264.
- 78.: Donis-Keller, H.. and V. Buckle. 1991. Report of the committee on the genetic constitution of chromosome 8. *Cytogen. Cell Genet.* 58: 382-402.
- 79.: Eidelmann, F. J.. A. Fuks, L. DeMarte. M. Taheri, and C. P. Stanners. 1993. Human carcinoembryonic antigen, an intercellular adhesion molecule, blocks fusion and differentiation of rat myoblasts. *J. Cell Biol.* 123:467-475.
- 80.: Feinberg, A. P., and B. Vogelstein, 1983. A technique for radiolabeling DNA restriction endonuclease fragments to high specific activity. *Anal. Biochem.* 132:6-13.
- 81.: Ferguson, M. A. J. 1988. Cell-surface anchoring of proteins via glycosyl-phosphatidylinositol structures. *Ann. Rev. Biochem.* 57:285-320.
- 82.: Fleming. T. J.. C. O'Huigin. and T. R. Malek. 1993. Characterization of two novel Ly-6 genes: protein sequence and potential structural similarity to α-bungarotoxin and other neurotoxins. *J. Immunol.* 150:5379-5390.
- 83.: Forsberg, U. H., V. Bazil, I. Stefanova, and J. Schroder. 1989. Gene for human CD59 (likely Ly-6 honologue) is located on the short arm of chromosome 11. *Immunogen.* 30:188-193.
- 84.: Fournie, J. J.. R. J. Mullins, and A. Basten. 1991. Isolation and structural characteristics of a monoclonal antibody-defined cross-reactive phospholipid antigen from *Mycobacterium* tuberculosis and *Mycobacterium leprae. J. Biol. Chem.* 266:1211-1219.
- 85.: Garcia, M., C. Mirre, A. Quaroni, H. Reggio, and A. Lebivic. 1993. CRG-anchored proteins associate to form microdomains during their intracellular transport in Caco-2 cells. *J. Cell. Sci.* 104:1281-1290.
- 86.: Gerretsen. M., J. J. Quak, J. S. Suh, M. van Walsum, C. J. L. M. Meijer, G. B. Snow, and G. A. M. S. van Dongen. 1991. Superior localisation of monoclonal antibody E48 F(ab')2 fragment in xenografts of human squamous cell carcinoma of the head and neck and of the vulva as compared to monoclonal antibody E48 IgG. *Br. J. Cancer* 63:37-44.
- 87.: Geiger, B., and O . Ayalon. 1992. Cadherins. Ann. *Rev. Cell Biol.* 8:307-332.
- 88.: Gorbsky, G., and S. M. Steinberg. 1981. Isolation of the intercellular glycoproteins of desmosomes. J. *Cell Biol.* 90, 243-248.
- 89.: Gordon, D. L., T. Sadlon. C. Hefford, and D. Adrian. 1993. Expression of CD59, a regulator of the membrane attack complex of complement, on human astrocytes. Brain Res. *Mol. Brain Res.* 18:335-338.
- 90.: Gough, N. M. 1988. Rapid and quantitative preparation of cytoplasmic RNA from small numbers of cells. *Anal. Biochem.* 173:93-95.
- 91.: Gumley. T. P., I. F. C. McKenzie. and M. S. Sandrin. 1994. Polymorphism at the mouse ThB locus. *Immunogen.* 36:390-394.
- 92.: Gumley, T. P., I. F. C. McKenzie, C. Kozak, and M. S. Sandrin. 1992. Isolation and characterization of cDNA clones for the mouse thymocyte B cell antigen (ThB). *J. Immunol*. 149:2615-2618.
- 93.: Havran. W. L., D. W. Lancki, R. L. Moldwin, D. P. Dialynas, and F. W. Fitch. 1988. Characterization of an anti-Ly-6 monoclonal antibody which defines and activates cytolytic T lymphocytes. *J. Immunol.* 140;1034-1042.
- 94.: Henderson, G. B., and E. M. Zevely. 1980. Transport of methotrexate in L1210 cells: effect of ions on the rate and extent of uptake. *Arch. Biochem. Biophys.* 200:149-155.
- 95.: Hogenesch, H., B. de Geus, F. Tielen, and J. Rozing. 1993. Constitutive expression of Ly-6A.2 on murine keratinocytes and inducible expression on TCR γδ+ dendritic epidermal T-cells. J. *Dermatol. Sci.* 5:114-121.
- 96.: Kamiura, S., C. M. Nolan, and D. Meruelo. 1992. Long-range physical map of the Ly-6 complex: mapping the ly-6 multigene family by field-inversion and two dimensional gel electrophoresis. *Genomics* 12:89-105.
- 97.: Korty, P. E., C. Brando, and E. M. Shevach. 1991. CD59 functions as a signal-transducing molecule for human T cell activation. *J. Immunol.* 146:4092-4098.
- 98.: Laemmli, U. K. 1970. Cleavage of structural proteins during the assembly of the head of bacteriophage T4. *Nature* 227:680-685.
- 99.: LeClair, K. P., R. G. E. Palfree, P. M. Flood, U. Hammerling, and A. Bothwell. 1986. Isolation of a murine Ly-6 cDNA reveals a new multigene family. *EMBO* J. 5:3227-3234.
- 100.: Luhrs. C. A., and B. L. Slomiany. 1989. A human membrane-associated folate binding protein is anchored by a glycosyl-phosphatidylinositol tail. *J. Biol. Chem.* 264:21446-21449.
- 101.: Malek, T. R., G. Ortega, C. Chan, R. A. Koczek, and E. M. Shevach. 1986. Role of Ly-6 in lymphocyte activation. II. Induction of T cell activation by monoclonal anti-Ly-6 antibodies. *J. Exp. Med.* 164:709-722.
- 102.: MacNeil. I., J. Kennedy, D. I. Godfrey, N. A. Jenkins, M. Masciantonio, C. Mineo. D. J. Gilbert, N. G. Copeland, R. L. Boyd, and A. Zlotnik. 1993. Isolation of a cDNA encoding thymic shared antigen-1. *J. Immunol. 151*:6913-6923.
- 103.: Mayor, S., K. G. Rothberg. and F. R. Maxfield. 1994. Sequestration of CRG-anchored proteins in caveolae triggered by cross-linking. *Science* 264:1948-1951.
- 104.: Palfree, R. G. E. 1991. Ly-6 nomenclature. *Immunol. Today* 12:45-46.
- 105.: Palfree, R. G. E., S. Sirlin, F. L. J. Dumont. and U. Hammerling. 1988. N-terminal and cDNA characterization of murine lymphocyte antigen Ly-6C.2. *J. Immunol.* 140:305-310.
- 106.: Proudfoot, N. J. 1989. How RNA polymerase II terminates transcription in higher eucaryotes. Trends *In Biochem. Sci.* 14:105-110.
- 107.: Proudfoot, N. J. 1991. Poly(A) signals. *Cell* 64:671-674.
- 108.: Quak, J. J., G. A. M. S. van Dongen, A. J. M. Balm. J. P. G. Brakkee. R. L. Scheper, G. B. Snow, and C. J. L. M. Meijer. 1990. A 22-kDa surface antigen detected by monoclonal antibody E48 is exclusively expressed in stratified squamous and transitional epithelia. *Am. J. Pathol.* 136:191-197.
- 109.: Ronmens, C. M. T., E. A. van der Biezen, P. B. F. Ouwerkerk, J. J. Nijkamp, and J. Hille. 1991. Ac-induced disruption of the double Ds structure in tomato. *Mol. Gen. Genet.* 228:453-458.
- 110.: Rothberg, K. G., J. E. Heuser, W. C. Donzell, Y.-S. Ying, J. R. Glenney, and R. G. W. Anderson. 1992. Caveolin, a protein component of caveolae membrane coats. *Cell* 68:673-682.
- 111.: Sambrook. J., E. F. Fritsch, and T. Maniatis. 1989. Molecular Cloning: a Laboratory Manual. Cold Spring Harbor Laboratory Press, Cold Spring Harbor, New York.
- 112.: Sargiacomo, M., M., Sudol, Z. Tang, and M. P. Lisanti. 1993. Signal transducing molecules and glycosyl-phosphatidylinositol-linked proteins form a caveolin-rich insoluble complex in MDCK cells. *J. Cell Biol.* 122:789-807.
- 113.: Schmidt, J. W., P. A. Piepenhagen. and W. J. Nelson. 1993. Modulation of epithelial morphogenesis and cell fate by cell-to-cell signals and regulated cell adhesion. *Mol. Cell. Biol.* 4:161-173.
- 114.: Schrijvers. A. H. G. J., M. Gerretsen, J. Fritz, M. van Walsum. J. J. Quak, G. B. Snow, and G. A. M. S. van Dongen. 1991. Evidence for a role of the monoclonal antibody E48 defined antigen in cell-cell adhesion in squamous epithelia and head and neck squamous cell carcinomas. *Exp. Cell Res.* 196:264-269.
- 115.: Schrijvers, A. H. G. J., M. Gerretsen, M. van Walsum, B. J. M. Braakhuis, J. J. Quak, G. B. Snow, and G. A. M. S. van Dongen. 1992. Potential for targeting head and neck squamous cell carcinoma with monoclonal antibody K984. *Cancer Immunol. Immunother.* 34:252-258.
- 116.: Schwarz, M. A., K. Owaribe, J. Kartenbeck, and W. W. Franke. 1990. Desmosomes and hemidesmosomes: constitutive molecular components. *Ann. Rev. Cell. Biol.* 6:461-491.
- 117.: Seed, B. 1987. An LFA-3 cDNA encodes a phospholipid-linked membrane protein to its receptor CD2. *Nature* 329:840-842.
- 118.: Shenoyscaria. A. M., J. Kwong. T. Fujita. M. W. Olszowy, A. S. Shaw, and D. M. Lublin. 1992. Signal transduction through decay accelerating factor. Interaction of glycosyl-phosphatidylinositol anchor and protein tyrosine kinases p56lck and p59fynl. *J. Immunol.* 149:3535-3541.
- 119.: Shenoyscaria, A. M., L. K. T. Gauen, J. Kwong. A. S. Shaw, and D. M. Lublin. 1993. Palmityolation of an amino-terminal cysteine motif of protein tyrosine kinases p56lck and p59fyn mediates interaction with glycosyl-phosphatidylinositol-anchored proteins. *Mol. Cell. Biol*. 13:6385-6392.
- 120: Stefanova, I., V. Horejsi. I. J. Ansotegui. W. Knapp, and H. Stockinger. 1991. CRG-anchored cell-surface molecules complexed to protein tyrosine kinases. *Science* 254:1016-1019.
- 121.: Su, L. K., B. Vogalstein, and K. W. Kinzler. 1993. Association of the APC tumor suppressor gene with catenins. *Science* 262:1734-1737.
- 122.: Suijkerbuijk, R. F., L. Looijenga, B. de Jong, J. W. Oosterhuis, J. J. Cassiman, and A. H. M. Geurts van Kessel. 1992. Verification of isochromosome 12p and identification of other chromosome 12 aberrations in gonadal and extragonadal human germ cell tumors by bicolor double fluorescence in situ hybridization. *Cancer Genet. Cell Genet.* 63:8-16.
- 123.: Takeichi. M. 1990. Cadherins: a molecular family important in selective cell-cell adhesion. *Ann. Rev. Biochem.* 59:237-252.
- 124.: Tone, M., L. A. Walsh, and H. Waldmann. 1992. Gene structure of human CD59 and demonstration that discrete mRNAs are generated by alternative polyadenylation. *J. Mol. Biol.* 227:971-976.
- 125.: Troyanovsky, S. M., L. G. Eshkind, R. B. Troyanovsky, R. E. Leube, and W. W. Franke. 1993. Contributions of cytoplasmic domains of desmosomal cadherins to desmosome assembly and intermediate filament anchorage. *Cell* 72:561-574.
- 126.: Tsukita, S., K. Oishi, T. Akiyama, Y. Yamashi, and T. Yamamoto. 1991. Specific proto-oncogenic tyrosine kinases of the src family are enriched in cell to cell adherens junctions where the level of tyrosine phosphorylation is elevated. *J. Cell Biol.* 113:867-879.
- 127.: Vleminckx, K., L. Jr. Vakaet. M. M. Mareel, W. Fiers, and F. van Roy. 1991. Genetic manipulation of E-cadherin expression by epithelial tumor cells reveals an invasion suppressor role. *Cell* 66:107-119.
- 128.: Wahle, E.. and W. Keller. 1992. The biochemistry of 3'-end cleavage and polyadenylation of messenger RNA precursors. *Ann. Rev. Biochem.* 61:419-440.
- 129.: Wheelock, M. J., and P. J. Jensen. 1992. Regulation of keratinocyte intercellular junction organization and epidermal morphogenesis by E-cadherin. *J. Cell Biol.* 117:415-425.
- 130.: Wickens, M. 1990. How the messenger got its tail: addition of poly(A) in the nucleus. *Trends in Biochem. Sci.* 15:277-281.
- 131.: Wijngaard, P. L. J., M. J. Metzelaar, N. D. MacHugh, W. I. Morrison, and H. C. Clevers. 1992. Molecular characterization of the WC1 antigen expressed specifically on bovine cd4-cd8-γδT lymphocytes. *J. Cell Biol.* 149:3273-3277.
- 132.: Zurzolo, C., W. van 't Hof. G. van Meer, and E. Rodriquez-Boulan. 1994. VIP21/caveolin, glycosphingolipid clusters and the sorting of glycosyl-phosphatidylinositol-anchored proteins in epithelial cells. *EMBO J.* 13:42-53.
- 133.: Zurzolo, C.. M. P. Lisanti, I. W. Caras, L. Nitsch, and E. Rodriguez-Boulan. 1993. Glycosyl-phosphatidylinositol-anchored proteins are preferentially targeted to the basolateral surface in fischer rat thyroid epithelial cells. *J. Cell Biol.* 121:1031-1039.
- 134.: E. Vokes et al.. The New England Journal of Medicine (Jan 21, 1993 p.184-194)
- 135.: Stamenkovic I et al 1989. Cell 56: 1057-1062
- 136.: Birch M. et al 1991, Cancer Res. 51:6660-6667
- 137.: Gunthert U. et al. Cell 65 1991: 13-24
- 138.: Sy M.S. et al. J. Exp. Med. 1991 174: 859-866
- 139.: Hofmann M. et al Cancer Res. 51 1991: 5292-5297
- 140.: Stamenkovic I. et al. EMBO J. 10 1991: 343-348
- 141.: Jackson D.G. et al J. Biol. Chem. 1992 267: 4732-4739
- 142.: Schlimok G. et al. 1990. Journal of Clinical Oncology, Vol 8, No 54, 1990: pp 831-837
- 143.: Schlimok G. and Rietmuller G. Seminars in Cancer Biology
- 144.: Smit B. et al. The Lancet 1991, vol. 338 Nov. 16:1227
- 145.: Moreno J.G. et al. Cancer Res. 52, 6110-6112, November 1. 1992
- 146.: Wollenberg B. et al., Laryngo-Rhino-otology 73 (1994) 88-93
- 147.: Said J.W. et al. Lab. Invest. 1983, 49:563-568
- 148.: Ramaekers F. et al. Exp. Cell Res 1987, 17 235-249
- 149.: Tobal et al. 1993
- 150.: Fynan E.F. Proc Natl. Acad. Sci. vol.90, pp 11478-11482, Dec.93
- 151.: Bakker A.B.; Scheurs W.J.; de Boer A.J.; Kawakami Y; Rosenberg S.A.; Adema G.J.; Figdor C.G. (1994). Melanocyte linage-specific antigen gp100 is recognized by melanoma derived tumor infiltrating lymphocytes J. Exp. Med. 179:1005-1009
- 152.: Greenhalgh D.A., Rothnagel J.A. and Roop D.R. J. Invest. Dermatol 103:63S-69S, 1994
- 153.: Lindemann F.; Schlimok G.; Dirschedel P.; Witte J.; Riethmuller G. (1992). Prognostic significance of micrometastatic tumor cells in bone marrow of colorectal cancer patients. Lancet 19: 685-689.
- 154.: Hogarth, P. M., I. F. C. McKenzie, V. R. Sutton, K. M. Curnow, B. K. Lee, and E. M. Eicher. 1987. Mapping of the Ly-6, Xp14 and Gdc-I loci to chromosome 15. *Immunogen*. 25:21-27
- 155.: Rock, K. L., H. Reiser, H. Bamezai, J. McGrew, and B. Benacerraf. 1989. The Ly-6 locus: a multigene family encoding phosphatidylinositol-anchored membrane proteins concerned with T-cell activation. *Immunol. Rev.* 3:195-224.
- 156.: Quak, J., Gerretsen, M., de Bree, R., Brakenhof, R., van Dongen, G. and Snow, G. (1993) Perspectives of monoclonal antibodies for detection and treatment of head and neck tumours. Anticancer Research 13:2533-2540.

Sequence ID.1

cDNA sequence encoding the E48 antigen (from nucleotide 24-408)

10 20 30 40 50 60

1 ACTGCTCCCAGACGACATCAGAGATGAGGACAGCATTGCTGCTCCTTGCAGCCCTGGCTG

61 TGGCTACAGGGCCAGCCCTTACCCTGCGCTGCCACGTGTGCACCAGCTCCAGCAACTGCA

121 AGCATTCTGTGGTCTGCCCGGCCAGCTCTCGCTTCTGCAAGACCACGAACACAGTGGAGC

181 CTCTGAGGGGGAATCTGGTGAAGAAGGACTGTGCGGAGTCGTGCACACCCAGCTACACCC

241 TGCAAGGCCAGGTCAGCAGCGGCACCAGCTCCACCCAGTGCTGCCAGGAGGACCTGTGCA

301 ATGAGAAGCTGCACAACGCTGCACCCACCCGCACCGCCCTCGCCCACAGTGCCCTCAGCC

361 TGGGGCTGGCCCTGAGCCTCCTGGCCGTCATCTTAGCCCCCAGCCTGTGACCTTCCCCCC

421 AGGGAAGGCCCCTCATGCCTTTCCTTCCCTTTCTCTGGGGATTCCACACCTCTCTTCCCC

481 AGCCGGCAACGGGGGTGCCAGGAGCCCCAGGCTGAGGGCTTCCCCGAAAGTCTGGACCA

541 GGTCCAGGTGGGCATGGAATGCTGATGACTTGGAGCAGGCCCCACAGACCCCACAGAGGA

601 TGAAGCCACCCCACAGAGGATGCAGCCCCCAGCTGCATGGAAGGTGGAGGACAGAAGCCC

661 TGTGGATCCCCGGATTTCACACTCCTTCTGTTTTGTTGCCGTTTATTTTGTACTCAAATC

721 TCTACATGGAGATAAATGATTTAAACCAAAAAAAAAAAAAAATCTAGAGTCGGAATTCC

Total number of bases is: 779

Sequence ID.2

Amino acid sequence of the E48 antigen, translated from the cDNA sequence
ID.1 from nucleotide 24 to 408.

10 20 30 40 50 60
| | | | | |
1 MRTALLLLAA LAVATGPALT LRCHVCTSSS NCKHSVVCPA SSRFCKTTNT VEPLRGNLVK
61 KDCAESCTPS YTLQGQVSSG TSSTQCCQED LCNEKLHNAA PTRTALAHSA LSLGLALSLL
121 AVILAPSL

Number of residues : 128
Molecular weight (MW) : 13286

Sequence ID.3

E48-gene - GGATCCCCGGCATCTGAGACGTGGGGCTCAGCAAGGGAGAGATGCCGGGA -50

E48-gene - GGAAGGGCCAGAGGGTCTGTCTCAAGCCCAGGCCTGACTGGNCCCAGTAC -100

E48-gene - CCACGGTGCTCCCAAAAGCCTCAGCCCAGCCCAGGACCACCCTCCTCGGA -150

E48-gene - ACCGGTCCTGTCCTGCCCTGGWCTGTTGTTGTCTGTTCATCAGCCACGT -200

E48-gene - GGCAGCGGWGGCAGGAGGCTGGGCCCAGGGWTGACCTCCATCTGTGCCCC -250

E48-gene - GGCTCCCACTCACCTTCAGCCTAGCCAGGGWAGGGGNAAGCAAGTCTGTG -300

E48-gene - GGCCCCTCCACACCACTCAGGTGGGTGCTGWCCAGCACAGGCGAGCGGNA -350

E48-gene - GAGCGTNCTGGGWTGAACAGGTGGATGGGGGT--1 kb-AGGATCCCGTC -400

E48-gene - CTGCGTGACGCTTCGACAGAGCCCTGCAGAATGCCGCTGGCTGGCGGACT -450

E48-gene - GCCCACCCCCGCCCAGCCCGTGCCTATAAGGCCTTGGCAATGCAGGGGCC -500

E48-gene - CGCACTGCTCCCAGACGACATCAGAGATGAGGACAGCATTGCTGCTCCTT -550
||||||||||||||||||||||||||||||||||||||||||||||
E48-cDNA - ACTGCTCCCAGACGACATCAGAGATGAGGACAGCATTGCTGCTCCTT -47

E48-gene - GCAACCCTGGCTGTGGCTACAGGGCCAGGTGAGGGGCTGGCCAAGGGGA -600
||| ||||||||||||||||||||||||
E48-cDNA - GCAGCCCTGGCTGTGGCTACAGGGCCAG---------------------- -76

E48-gene - CGGGCGGGTGGGCGGCTCTGGCCTGTGTGGTCACCAAATACAGGAGCCTG -650

E48-cDNA - -------------------------------------------------- -76

E48-gene - GAGCTGAGCACATCTAGGCACAGAGAACCCCGACACTGTGGGTGNTAGAC -700

E48-cDNA - -------------------------------------------------- -76

E48-gene - TCTTCAANTTTNAAACAACATGGAGCCCGAATCCTTAACGAGTGTCAGAT -750

E48-cDNA - -------------------------------------------------- -76

E48-gene - TTACAATGTTTCAGAATCTGAGGATCTTAGAACTGACTCCTGGAGTCACG -800

E48-cDNA - -------------------------------------------------- -76

E48-gene - GACCCTGAGAGAGTGGAGCAGGGGGTTTGTCTTGGAGATGCTT-50 bp- -850

E48-cDNA - -------------------------------------------------- -76

E48-gene - -GCCCCTCCCGGTCCCCAGGTCTCCCCCCAGCACAGCCCAGGACAGCCCA -900

E48-cDNA - -------------------------------------------------- -76

E48-gene - AAGGCCACCAAGCCTTCCTGTCATAGGCTCAGAAACAGCCCAAGCTGGCT -950

E48-cDNA - -------------------------------------------------- -76

E48-gene - GCCCCCGGGACCTTAGCTCCGTTCAGGCCCTCAGAAACATAGCCATCTCC -1000
E48-cDNA - -------------------------------------------------- -76
E48-gene - TCCAGGGACATCCCCTGGGGTGGGGACAAGAGCCAGTGTCCTCTTGACAG -1050
E48-cDNA - -------------------------------------------------- -76
E48-gene - TCACAGAGCTCCCTGAGACCTTGGAGGAAAGGCTTGCAACACACCCTAGA -1100
E48-cDNA - -------------------------------------------------- -76
E48-gene - CAGGGGCAGGGCAGGACACCTCTTCCAAGCCGGCCCAGGCTGGAATACAT -1150
E48-cDNA - -------------------------------------------------- -76
E48-gene - GTCCCCTGAGGCTGCACTCAGGCACATGCCCTGCCAGACCCAGAGCCCGG -1200
E48-cDNA - -------------------------------------------------- -76
E48-gene - GTTTGGGGGCCTAGGGCAAGGGCAGCAGGGCCTCTGTCCAGGAGGGGTCT -1250
E48-cDNA - -------------------------------------------------- -76
E48-gene - CTGCAGGTGGGGGAGCAGGCCCTGCTAAGTCACCAGGAGGCCTCTGTTGG -1300
E48-cDNA - -------------------------------------------------- -76
E48-gene - GCCCGCTGAGCCGGACTCCCTCCCCGCCAGCCCTTACCCTGCGCTGCCAC -1350
||||||||||||||||||||
E48-cDNA - ------------------------------CCCTTACCCTGCGCTGCCAC -95
E48-gene - GTGTGCACCAGCTCCAGCAACTGCAAGCATTCTGTGGTCTGCCCGGCCAG -1400
||||||||||||||||||||||||||||||||||||||||||||||||||
E48-cDNA - GTGTGCACCAGCTCCAGCAACTGCAAGCATTCTGTGGTCTGCCCGGCCAG -145
E48-gene - CTCTCGCTTCTGCAAGACCACGAACACAGGTGGGTAGCATCTGTCCAGGT -1450
||||||||||||||||||||||||||||||
E48-cDNA - CTCTCGCTTCTGCAAGACCACGAACACAG--------------------- -175
E48-gene - GTCCCTGGGGCTGGGGACATCCCTGGCTGTTGTGGGTGTTGGCTGGAAGG -1500
E48-cDNA - -------------------------------------------------- -175
E48-gene - TTGTGGGGAGGTGGGGGGCAGAGGTGGCTGCCTGGCCTGACCACACTCAC -1550
E48-cDNA - -------------------------------------------------- -175
E48-gene - CTGTGCCCAGTGGAGCCTCTGAGGGGGAATCTGGTGAAGAAGGACTGTGC -1600
|||||||||||||||||||||||||||||||||||||||
E48-cDNA - ----------TGGAGCCTCTGAGGGGGAATCTGGTGAAGAAGGACTGTGC -214
E48-gene - GGAGTCGTGCACACCCAGCTACACCCTGCAAGGCCAGGTCAGCAGCGGCA -1650
||||||||||||||||||||||||||||||||||||||||||||||||||
E48-cDNA - GGAGTCGTGCACACCCAGCTACACCCTGCAAGGCCAGGTCAGCAGCGGCA -264

E48-gene - CCAGCTCCACCCAGTGCTGCCAGGAGGACCTGTGCAATGAGAAGCTGCAC -1700
E48-cDNA - CCAGCTCCACCCAGTGCTGCCAGGAGGACCTGTGCAATGAGAAGCTGCAC -314

E48-gene - AACGCTGCACCCACCCGCACCGCCCTCGCCCACAGTGCCCTCAGCCTGGG -1750
E48-cDNA - AACGCTGCACCCACCCGCACCGCCCTCGCCCACAGTGCCCTCAGCCTGGG -364

E48-gene - GCTGGCCCTGAGCCTCCTGGCCGTCATCTTAGCCCCCAGCCTGTGACCTT -1800
E48-cDNA - GCTGGCCCTGAGCCTCCTGGCCGTCATCTTAGCCCCCAGCCTGTGACCTT -414

E48-gene - CCCCCCAGGGAAGGCCCCTCATGCCTTTCCTTCCCTTTCTCTGGGGATTC -1850
E48-cDNA - CCCCCCAGGGAAGGCCCCTCATGCCTTTCCTTCCCTTTCTCTGGGGATTC -464

E48-gene - CACACCTCTCTTCCCCAGCCGGCAACGGGGGTGCCAGGAGCCCCAGGCTG -1900
E48-cDNA - CACACCTCTCTTCCCCAGCCGGCAACGGGGGTGCCAGGAGCCCCAGGCTG -514

E48-gene - AGGGCTTCCCCGAAAGTCTGGGACCAGGTCCAGGTGGGCATGGAATGCTG -1950
E48-cDNA - AGGGCTTCCCCGAAAGTCTGGGACCAGGTCCAGGTGGGCATGGAATGCTG -564

E48-gene - ATGACTTGGAGCAGGCCCCACAGACCCCACAGAGGATGAAGCCACCCCAC -2000
E48-cDNA - ATGACTTGGAGCAGGCCCCACAGACCCCACAGAGGATGAAGCCACCCCAC -614

E48-gene - AGAGGATGCAGCCCCCAGCTGCATGGAAGGTGGAGGACAGAAGCCCTGTG -2050
E48-cDNA - AGAGGATGCAGCCCCCAGCTGCATGGAAGGTGGAGGACAGAAGCCCTGTG -664

E48-gene - GATCCCCGGATTTCACACTCCTTCTGTTTTGTTGCCGTTTATTTTGTACT -2055
E48-cDNA - GATCCCCGGATTTCACACTCCTTCTGTTTTGTTGCCGTTTATTTTGTACT -714

E48-gene - CAATTCTCTACATGGAGATAAA
E48-cDNA - CAAATCTCTACATGGAGATAAATGATTTAAACCAAAAAAAAAAAAAAATC -764

E48-cDNA - TAGAGTCGGAATTCC -779

## Claims

1. A method for detection of squamous cell carcinoma and bladder carcinoma in a sample, said sample preferably being of human origin, said method comprising detection of expression of a nucleic acid encoding a glycosyl-phosphatidylinositol (= GPI)-anchored membrane protein, wherein the GPI-anchored membrane protein belongs to the family of human proteins having a molecular weight below 30 kDa as determined by SDS-PAGE under non-reducing conditions comprising a cysteine-rich amino acid sequence with 10 cysteines in a contiguous amino acid sequence of 138 amino acids or less, said family to be referred to as human Cysteine-Rich GPI-anchored proteins (=human CRG proteins), said family of proteins preferably comprising the following consensus CX₁₋₅CX₅₋₁₅CX₃₋₈CX₃₋₈CX₁₀₋₃₀CX₂₋₇CX₁₀₋₃₀CCX₁₋₈CN, more preferably CX₁₋₅CX₅₋₁₅CX₃₋₈CX₃₋₈CX₁₀₋₃₀CX₂₋₇CX₁₀₋₃₀CCX₁₋₆DLCN and most preferably CX₂CX₉CX₄ₒᵣ₅CX₆CX₁₂₋₂₂CX₃₋₅CX₁₆₋₁₉CCX₂₋₄DLCN, in particular the consensus CX₂CX₉CX₄ₒᵣ₅CX₆CX₁₂₋₂₂CX₃₋₅CX₁₆₋₁₉CCX₂DLCN and most preferably the consensus CX₂CX₅CX₅CX₆CX₁₇CX₃CX₁₈CCX₂DLCN, wherein X can be any amino acid, C is cysteine, D is aspartic acid, L is leucine and N is asparagine.

2. A method according to any of the preceding claims wherein the consensus sequence comprises LX₁₋₃CX₁₋₅CX₅₋₁₅CX₃₋₈CX₃₋₈CX₁₀₋₃₀CX₂₋₇CX₁₀₋₃₀CCX₁₋₈CN, more preferably LX₁₋₃CX₁₋₅CX₅₋₁₅CX₃₋₈CX₃₋₈CX₁₀₋₃₀CX₂₋₇CX₁₀₋₃₀CCX₁₋₆DLCN, most preferably LXCX₂CX₉CX₄ₒᵣ₅CX₆CX₁₂₋₂₂CX₃₋₅CX₁₆₋₁₉CCX₂₋₄DLCN, in particular the consensus LXCX₂CX₉CX₄ₒᵣ₅CX₆CX₁₂₋₂₂CX₃₋₅CX₁₆₋₁₉CCX₂DLCN and most preferably the consensus LXCX₂CX₅CX₅CX₆CX₁₇CX₃CX₁₈CCX₂DLCN, wherein X can be any amino acid, C is cysteine, D is aspartic acid, L is leucine and N is asparagine.

3. A method according to claim 1 or 2, wherein the sample is a sample normally free of mRNA encoding the CRG protein, such a sample for example being bone marrow, lymph nodes or peripheral blood.

4. A method according to any of the preceding claims, wherein the genomic sequence encoding the CRG protein is located on human chromosome 8 in the 8q24-qter region.

5. A method according to any of the preceding claims, wherein the CRG protein belongs to the family of human Ly-6 proteins, said protein preferably exhibiting more than 50% homology with the amino acid sequence of sequence id no 2, preferably more than 66%, most preferably 90-100%.

6. A method according to any of the preceding claims, wherein the nucleic acid sequence to be detected comprises a nucleic acid sequence according to any of the claims 18-24.

7. A method according to any of the preceding claims, wherein the nucleic acid sequence to be detected is a nucleic acid sequence encoding the amino acid sequence according to sequence id no 2.

8. A method according to any of the preceding claims, wherein the nucleic acid sequence to be detected is a nucleic acid sequence according to sequence id no 1.

9. A method according to any of the preceding claims, wherein the method for detection comprises use of a molecule capable of specifically binding to a part of the nucleic acid sequence to be detected or the complementary sequence thereof.

10. A method according to claim 9, wherein the molecule is a nucleic acid sequence of at least ten nucleotides capable of specifically binding to a part of the nucleic acid sequence to be detected or the complementary sequence thereof under normal to stringent hybridisation conditions.

11. A method according to claim 9 or 10, wherein the method for nucleic acid detection comprises nucleic acid amplification with one or more primers followed by detection of the amplified nucleic acid with a probe, said method optionally including immobilisation of the amplified nucleic acid sequence and detection of the immobilised amplified nucleic acid sequence, wherein said probe or primer can be a molecule as described in claim 10.

12. A method according to claim 11, wherein a part of the nucleic acid sequence to be detected is amplified with the resulting amplified part preferably comprising an exon-exon junction as can be achieved by appropriate selection of primers for the amplification reaction, such as a sense primer of exon 1 of the nucleic acid sequence according to sequence id no. 1 and 3 and an antisense primer of exon 2 or 3 of the nucleic acid sequence according to sequence id no. 1 and 3.

13. A method for determining the presence of minimal residual disease, micrometastases or dissemination of the squamous carcinoma type or the bladder carcinoma type, comprising analysis of a sample, preferably a human sample, said sample not normally comprising keratinocytes or epithelial cells said sample for example being of lymph node tissue, blood or bone marrow, said method comprising carrying out one of the methods according to any of the preceding claims, wherein detection of the nucleic acid sequence in the sample is indicative of minimal residual disease, micrometastases or tumor cell dissemination.

14. A method according to claim 13, wherein the sample to be tested is blood or bone marrow that has been subjected to purging of squamous carcinoma type cells and bladder carcinoma type cells therefrom.

15. A pharmaceutical composition comprising an expression vector, suitable for expressing nucleic acid in the patient, preferably being suitable for humans, said expression vector expressing a nucleic acid sequence encoding a CRG protein as defined in claims 1-5 as active component in a pharmaceutically effective amount, a pharmaceutically acceptable carrier and optionally an adjuvant.

16. An isolated or recombinant nucleic acid sequence encoding the amino acid sequence of sequence id no 2 or being the complementary nucleic acid sequence thereof.

17. An isolated or recombinant nucleic acid sequence according to sequence id no 1 or the complementary sequence of sequence id. no. 1.

18. An isolated or recombinant nucleic acid sequence encoding a CRG protein and capable of hybridising to a nucleic acid sequence according to claim 16 or 17 under normal to stringent hybridisation conditions, wherein normal hybridisation conditions comprise hybridisation at T=55 °C, with 6xSSC and rinsing at T=55 °C, with 6xSSC and wherein stringent hybridisation conditions comprise hybridisation at T=65 °C, with 6xSSC and rinsing with 0,2xSSC at T=65 °C.

19. An isolated or recombinant nucleic acid sequence encoding a CRG protein and capable of hybridising to a nucleic acid sequence according to claim 18 under stringent hybridisation conditions .

20. An isolated or recombinant nucleic acid sequence according to any of claims 16-19, wherein the nucleic acid sequence encodes a consensus sequence as defined in claim 1 or 2.

21. An isolated or recombinant nucleic acid sequence according to claim 20, wherein the nucleic acid sequence encodes the amino acids 21-94 of the amino acid sequence of sequence id.no.2.

22. A part of the isolated or recombinant nucleic acid sequence according to sequence id no 3, wherein the part comprises the promoter sequence of the gene encoding the hCRG protein of amino acid sequence id no 2, i.e. nucleic acid sequence of nucleotide 1 to the first nucleotide of exon 1 according to sequence id no 1, i.e. the part between nucleotides 1-495 (= the Apa I site) of sequence id no 3.

23. A method of obtaining antibody specific for a marker of squamous cell carcinoma and bladder cell carcinoma in a sample not normally comprising keratinocytes or epithelial cells, said method comprising using an isolated or recombinant CRG protein as defined in claims 1-5 as an immunogen for antibody production in a test animal and obtaining the subsequently produced antibody from the test animal.

24. The method of claim 23 further comprising the step of fusing the cells which produce antibody against a CRG protein as defined in claims 1-5 with cancer cells to generate the hybridoma cells.

## Patentansprüche

1. Verfahren zum Plattenepithelkarzinom- und Blasenkarzinom-Nachweis in einer Probe, wobei die Probe vorzugsweise humanen Ursprungs ist, welches Verfahren den Nachweis der Expression einer Nukleinsäure umfasst, die für ein Glycosylphosphatidylinosit (= GPI)-verankertes Membranprotein kodiert, wobei das GPI-verankerte Membranprotein zur Familie von Humanproteinen mit einem Molekulargewicht unter 30 kD, wie mittels SDS-PAGE unter nicht reduzierenden Bedingungen bestimmt, gehört, die eine cysteinreiche Aminosäuresequenz mit 10 Cysteinen in einer zusammenhängenden Aminosäuresequenz von 138 Aminosäuren oder weniger umfassen, welche Familie als humane cysteinreiche GPI-verankerte Proteine (= humane CRG-Proteine) zu bezeichnen ist, wobei diese Familie von Proteinen vorzugsweise den folgenden Konsensus CX₁₋₅CX₅₋₁₅CX₃₋₈CX₃₋₈CX₁₀₋₃₀CX₂₋₇CX₁₀₋₃₀CCX₁₋₈CN, bevorzugter CX₁₋₅CX₅₋₁₅CX₃₋₈CX₃₋₈CX₁₀₋₃₀CX₂₋₇CX₁₀₋₃₀CCX₁₋₆DLCN und am meisten bevorzugt CX₂CX₉CX_{4oder5}CX₆CX₁₂₋₂₂CX₃₋₅CX₁₆₋₁₉CCX₂₋₄DLCN, insbesondere den Konsensus CX₂CX₉CX_{4oder5}CX₆CX₁₂₋₂₂CX₃₋₅CX₁₆₋₁₉CCX₂DLCN und am meisten bevorzugt den Konsensus CX₂CX₅CX₅CX₆CX₁₇CX₃CX₁₈CCX₂DLCN umfasst, wobei X jede Aminosäure sein kann, C Cystein ist, D Asparaginsäure ist, L Leucin ist und N Asparagin ist.

2. Verfahren nach irgendeinem der vorhergehenden Ansprüche, in dem die Konsensussequenz LX₁₋₃CX₁₋₅CX₅₋₁₅CX₃₋₈CX₃₋₈CX₁₀₋₃₀CX₂₋₇CX₁₀₋₃₀CCX₁₋₈CN, bevorzugter LX₁₋₃CX₁₋₅CX₅₋₁₅CX₃₋₈CX₃₋₈CX₁₀₋₃₀CX₂₋₇CX₁₀₋₃₀CCX₁₋₆DLCN, am meisten bevorzugt LXCX₂CX₉CX_{4oder5}CX₆CX₁₂₋₂₂CX₃₋₅CX₁₈₋₁₉CCX₂₋₄DLCN, insbesondere den Konsensus LXCX₂CX₉CX_{4oder5}CX₆CX₁₂₋₂₂CX₃₋₅CX₁₆₋₁₉CCX₂DLCN und am meisten bevorzugt den Konsensus LXCX₂CX₅CX₅CX₆CX₁₇CX₃CX₁₈CCX₂DLCN umfasst, wobei X jede Aminosäure sein kann, C Cystein ist, D Asparaginsäure ist, L Leucin ist und N Asparagin ist.

3. Verfahren nach Anspruch 1 oder 2, in dem die Probe eine Probe ist, welche normalerweise frei von mRNA ist, die für das CRG-Protein kodiert, wobei eine solche Probe beispielsweise Knochenmark, Lymphknotenmaterial oder peripheres Blut ist.

4. Verfahren nach irgendeinem der vorhergehenden Ansprüche, in dem die genomische Sequenz, die für das CRG-Protein kodiert, sich auf dem humanen Chromosom 8 in der 8q24-qter-Region befindet.

5. Verfahren nach irgendeinem der vorhergehenden Ansprüche, in dem das CRG-Protein zu der Familie von humanen Ly-6-Proteinen gehört, wobei das Protein vorzugsweise mehr als 50 % Homologie zu der Aminosäuresequenz von Sequenz-ID-Nr. 2, vorzugsweise mehr als 66 %, am meisten bevorzugt 90-100 %, aufweist.

6. Verfahren nach irgendeinem der vorhergehenden Ansprüche, in dem die nachzuweisende Nukleinsäuresequenz eine Nukleinsäuresequenz nach irgendeinem der Ansprüche 18-24 umfasst.

7. Verfahren nach irgendeinem der vorhergehenden Ansprüche, in dem die nachzuweisende Nukleinsäuresequenz eine Nukleinsäuresequenz ist, welche für die Aminosäuresequenz gemäß Sequenz-ID-Nr. 2 kodiert.

8. Verfahren nach irgendeinem der vorhergehenden Ansprüche, in dem die nachzuweisende Nukleinsäuresequenz eine Nukleinsäuresequenz gemäß Sequenz-ID-Nr. 1 ist.

9. Verfahren nach irgendeinem der vorhergehenden Ansprüche, in dem das Nachweisverfahren die Verwendung eines Moleküls umfasst, welches imstande ist, spezifisch an einen Teil der nachzuweisenden Nukleinsäuresequenz oder der komplementären Sequenz davon zu binden.

10. Verfahren nach Anspruch 9, in dem das Molekül eine Nukleinsäuresequenz von mindestens 10 Nukleotiden ist, welche imstande ist, spezifisch an einen Teil der nachzuweisenden Nukleinsäuresequenz oder der komplementären Sequenz davon unter normalen bis stringenten Hybridisierungsbedingungen zu binden.

11. Verfahren nach Anspruch 9 oder 10, in dem das Verfahren für den Nukleinsäurenachweis eine Nukleinsäureamplifizierung mit einem oder mehreren Primern, gefolgt vom Nachweis der amplifizierten Nukleinsäure mit einer Sonde, umfasst, wobei das Verfahren gegebenenfalls die Immobilisierung der amplifizierten Nukleinsäuresequenz und den Nachweis der immobilisierten amplifizierten Nukleinsäuresequenz einschliesst, wobei die Sonde oder der Primer ein Molekül wie in Anspruch 10 beschrieben sein kann.

12. Verfahren nach Anspruch 11, in dem ein Teil der nachzuweisenden Nukleinsäuresequenz amplifiziert wird, wobei der resultierende amplifizierte Teil vorzugsweise eine Exon-Exon-Verbindungsstelle umfasst, wie durch die geeignete Wahl von Primern für die Amplifizierungsreaktion, z.B. ein Sense-Primer von Exon 1 der Nukleinsäuresequenz gemäß Sequenz-ID-Nr. 1 und 3 und ein Antisense-Primer von Exon 2 oder 3 der Nukleinsäuresequenz gemäß Sequenz-ID-Nr. 1 und 3, erreicht werden kann.

13. Verfahren zur Feststellung des Vorhandenseins einer minimalen Resterkrankung, von Mikrometastasen oder Disseminierung vom Plattenepithelkarzinom- oder Blasenkarzinom-Typ, umfassend die Analyse einer Probe, vorzugsweise einer humanen Probe, welche Probe normalerweise keine Keratinozyten oder Epithelzellen umfasst, wobei die Probe beispielsweise von Lymphknotengewebe, Blut oder Knochenmark stammt, welches Verfahren die Durchführung eines der Verfahren nach irgendeinem der vorhergehenden Ansprüche umfasst, wobei der Nachweis der Nukleinsäuresequenz in der Probe eine minimale Resterkrankung, Mikrometastasen oder Tumorzelldisseminierung anzeigt.

14. Verfahren nach Anspruch 13, in dem die zu testende Probe Blut oder Knochenmark ist, das einer Entfernung von Zellen vom Plattenepithelkarzinom- und Blasenkarzinom-Typ daraus unterworfen wurde.

15. Pharmazeutische Zusammensetzung, umfassend einen Expressionsvektor, der zur Expression von Nukleinsäure in dem Patienten geeignet ist, vorzugsweise für Menschen geeignet ist, wobei der Expressionvektor eine Nukleinsäuresequenz exprimiert, die für ein CRG-Protein wie in den Ansprüchen 1 bis 5 definiert kodiert, als aktive Komponente in einer pharmazeutisch wirksamen Menge, einen pharmazeutisch annehmbaren Träger und gegebenenfalls ein Adjuvans.

16. Isolierte oder rekombinante Nukleinsäuresequenz, welche für die Aminosäuresequenz von Sequenz-ID-Nr. 2 kodiert oder die komplementäre Nukleinsäuresequenz davon ist.

17. Isolierte oder rekombinante Nukleinsäuresequenz nach Sequenz-ID-Nr. 1 oder die komplementäre Sequenz von Sequenz-ID-Nr. 1.

18. Isolierte oder rekombinante Nukleinsäuresequenz, die für ein CRG-Protein kodiert und imstande ist, mit einer Nukleinsäuresequenz nach Anspruch 16 oder 17 unter normalen bis stringenten Hybridisierungsbedingungen zu hybridisieren, wobei normale Hybridisierungsbedingungen eine Hybridisierung bei T=55°C mit 6xSSC und Spülen bei T=55°C mit 6xSSC umfassen und wobei stringente Hybridisierungsbedingungen eine Hybridisierung bei T=65°C mit 6xSSC und Spülen mit 0,2xSSC bei T=65° C umfassen.

19. Isolierte oder rekombinante Nukleinsäuresequenz, die für ein CRG-Protein kodiert und imstande ist, mit einer Nukleinsäuresequenz nach Anspruch 18 unter stringenten Hybridisierungsbedingungen zu hybridisieren.

20. Isolierte oder rekombinante Nukleinsäuresequenz nach irgendeinem der Ansprüche 16-19, wobei die Nukleinsäuresequenz für eine Konsensussequenz wie in Anspruch 1 oder 2 definiert kodiert.

21. Isolierte oder rekombinante Nukleinsäuresequenz nach Anspruch 20, wobei die Nukleinsäuresequenz für die Aminosäuren 21-94 der Aminosäuresequenz von Sequenz-ID-Nr. 2 kodiert.

22. Teil der isolierten oder rekombinanten Nukleinsäuresequenz gemäß Sequenz-ID-Nr. 3, wobei der Teil die Promotorsequenz des Gens umfasst, welches für das hCRG-Protein von Aminosäuresequenz-ID-Nr. 2 kodiert, d.h., die Nukleinsäuresequenz von Nukleotid 1 bis zum ersten Nukleotid von Exon 1 gemäß Sequenz-ID-Nr. 1, d.h., den Teil zwischen den Nukleotiden 1-495 (= der Apa I-Stelle) von Sequenz-ID-Nr. 3.

23. Verfahren zum Erhalt eines Antikörpers, der für einen Plattenepithelkarzinom- und Blasenzellkarzinom-Marker in einer Probe, die normalerweise keine Keratinozyten oder Epithelzellen umfasst, spezifisch ist, welches Verfahren die Verwendung eines isolierten oder rekombinanten CRG-Proteins wie in den Ansprüchen 1-5 definiert als Immunogen zur Antikörperproduktion in einem Versuchstier und Gewinnung des anschließend produzierten Antikörpers von dem Versuchstier umfasst.

24. Verfahren nach Anspruch 23, welches ferner den Schritt der Fusion der Zellen, welche einen Antikörper gegen ein CRG-Protein wie in den Ansprüchen 1-5 definiert produzieren, mit Krebszellen zur Erzeugung der Hybridomzellen umfasst.

## Revendications

1. Procédé pour la détection d'épithélioma spinocellulaire et d'épithélioma de la vessie dans un échantillon, ledit échantillon étant de préférence d'origine humaine, ledit procédé comprenant la détection d'une expression d'acide nucléique codant une protéine de membrane ancrée dans du glycosyl-phosphatidylinositol (= GPI), dans lequel la protéine de membrane ancrée dans du GPI appartient à la famille des protéines humaines possédant une masse moléculaire inférieure à 30 kDa comme cela est déterminé par le SDS-PAGE dans des conditions de non réduction comprenant une séquence d'acide aminé riche en cystéine avec 10 cystéines dans une séquence contiguë d'acide aminé de 138 acides aminés au maximum, ladite famille étant désignée "protéines ancrées dans du GPI riche en cystéine" (= protéines de CRG humain), ladite famille de protéines comprenant de préférence le consensus suivant CX₁₋₅CX₅₋₁₅CX₃₋₈CX₃₋₈CX₁₀₋₃₀CX₂₋₇CX₁₀₋₃₀CCX₁₋₈CN, et mieux CX₁₋₅CX₅₋₁₅CX₃₋₈CX₃₋₈CX₁₀₋₃₀CX₂₋₇CX₁₀₋₃₀CCX₁₋₆DLCN et encore mieux CX₂CX₉CX₄ₒᵤ₅CX₆CX₁₂₋₂₂CX₃₋₅CX₁₆₋₁₉CCX₂₋₄DLCN, plus particulièrement le consensus CX₂CX₉CX₄ₒᵤ₅CX₆CX₁₂₋₂₂CX₃₋₅CX₁₆₋₁₉CCX₂DLCN et encore mieux le consensus CX₂CX₅CX₅CX₆CX₁₇CX₃CX₁₈CCX₂DLCN, dans lequel X peut être n'importe quel acide aminé. C est de la cystéine, D est de l'acide aspartique, L est de la leucine et N est de l'asparagine.

2. Procédé selon l'une quelconque des revendications précédentes, dans lequel la séquence de consensus comprend LX₁₋₃CX₁₋₅CX₅₋₁₅CX₃₋₈CX₃₋₈CX₁₀₋₃₀CX₂₋₇CX₁₀₋₃₀CCX₁₋₈CN, et mieux LX₁₋₃CX₁₋₅CX₅₋₁₅CX₃₋₈CX₃₋₈CX₁₀₋₃₀CX₂₋₇CX₁₀₋₃₀CCX₁₋₆DLCN, encore mieux LXCX₂CX₉CX₄ₒᵤ₅CX₆CX₁₂₋₂₂CX₃₋₅CX₁₆₋₁₉CCX₂₋₄DLCN, plus particulièrement le consensus LXCX₂CX₉CX₄ₒᵤ₅CX₈CX₁₂₋₂₂CX₃₋₅CX₁₆₋₁₉CCX₂DLCN et encore mieux le consensus LXCX₂CX₅CX₅CX₆CX₁₇CX₃CX₁₈CCX₂DLCN, dans lequel X peut être n'importe quel acide aminé, C est de la cystéine, D est de l'acide aspartique, L est de la leucine et N est de l'asparagine.

3. Procédé selon la revendication 1 ou 2, dans lequel l'échantillon est un échantillon normalement exempt de ARNm codant la protéine de CRG, ledit échantillon étant par exemple de la moelle osseuse, des ganglions lymphatiques ou du sang périphérique.

4. Procédé selon l'une quelconque des revendications précédentes, dans lequel la séquence génomique codant la protéine de CRG est située sur le chromosome humain 8 dans la région 8q24-qter.

5. Procédé selon l'une quelconque des revendications précédentes, dans lequel la protéine de CRG appartient à la famille des protéines humaines Ly-6, ladite protéine démontrant de préférence plus de 50% d'homologie avec la séquence d'acide aminé de la séquence n°2, de préférence plus de 66% et encore mieux entre 90 et 100%.

6. Procédé selon l'une quelconque des revendications précédentes, dans lequel la séquence d'acide nucléique à détecter comprend une séquence d'acide nucléique selon l'une quelconque des revendications 18 à 24.

7. Procédé selon l'une quelconque des revendications précédentes, dans lequel la séquence d'acide nucléique à détecter est une séquence d'acide nucléique codant la séquence d'acide aminé selon la séquence n°2.

8. Procédé selon l'une quelconque des revendications précédentes, dans lequel la séquence d'acide nucléique à détecter est une séquence d'acide nucléique selon la séquence n°1.

9. Procédé selon l'une quelconque des revendications précédentes, dans lequel le procédé pour la détection comprend l'utilisation d'une molécule capable de se lier spécifiquement à une partie de la séquence d'acide nucléique à détecter ou à la séquence complémentaire de ladite séquence.

10. Procédé selon la revendication 9, dans lequel la molécule est une séquence d'acide nucléique d'au moins dix nucléotides capables de se lier spécifiquement à une partie de la séquence d'acide nucléique à détecter ou à la séquence complémentaire de ladite séquence dans des conditions d'hybridation normales à difficiles.

11. Procédé selon la revendication 9 ou 10, dans lequel le procédé pour la détection d'acide nucléique" comprend l'amplification de l'acide nucléique avec une ou plusieurs amorces suivie de la détection de l'acide nucléique amplifié à l'aide d'une sonde, ledit procédé comprenant optionnellement l'immobilisation de la séquence d'acide nucléique amplifié et la détection de la séquence d'acide nucléique amplifié immobilisé, dans lequel ladite sonde ou ladite amorce peuvent être une molécule telle que décrite dans la revendication 10.

12. Procédé selon la revendication 11, dans lequel une partie de la séquence d'acide nucléique à détecter est amplifiée avec la partie amplifiée résultante comprenant de préférence une jonction exon-exon pouvant être obtenue par la sélection appropriée d'amorces pour la réaction d'amplification, telles qu'une amorce de détection d'exon 1 de la séquence d'acide nucléique selon les séquences n°1 et 3 et une amorce antisens d'exon 2 ou 3 de la séquence d'acide nucléique selon les séquences n° 1 et 3.

13. Procédé pour déterminer la présence de maladie résiduelle minimale, de micrométastases ou de dissémination de type épithélioma squameux ou de type épithélioma vésical, comprenant l'analyse d'un échantillon, de préférence d'un échantillon humain, ledit échantillon ne comprenant normalement pas de kératinocytes ou de cellules épithéliales, ledit échantillon étant par exemple un tissu de ganglion lymphatique, du sang ou de la moelle osseuse, ledit procédé comprenant la réalisation de l'un des procédés selon l'une quelconque des revendications précédentes, dans lequel la détection de la séquence d'acide nucléique dans l'échantillon est indicative d'une maladie résiduelle minimale, de micrométastases ou d'une dissémination de tumeur oncogène.

14. Procédé selon la revendication 13, dans lequel l'échantillon à tester est du sang ou de la moelle osseuse qui a été soumis(e) à la purge des cellules de type épithélloma squameux et des cellules de type épithélioma vésical dudit sang ou de ladite moelle osseuse.

15. Composition pharmaceutique comprenant un vecteur d'expression, convenant à l'expression de l'acide nucléique chez le patient, convenant de préférence aux humains, ledit vecteur d'expression exprimant une séquence d'acide nucléique codant une protéine de CRG telle que définie dans les revendications 1 à 5 en tant que composant actif dans une quantité pharmaceutiquement efficace, en tant que porteur pharmaceutiquement acceptable et optionnellement en tant qu'adjuvant.

16. Séquence d'acide nucléique isolé ou recombinant codant la séquence d'acide aminé de la séquence n° 2 ou étant la séquence d'acide nucléique complémentaire de ladite séquence.

17. Séquence d'acide nucléique isolé ou recombinant selon la séquence n° 1 ou la séquence complémentaire de la séquence n°1.

18. Séquence d'acide nucléique isolé ou recombinant codant une protéine de CRG et étant capable d'hybrider une séquence d'acide nucléique selon la revendication 16 ou 17 dans des conditions d'hybridation normales à difficiles, dans laquelle les conditions normales d'hybridation comprennent l'hybridation à T=55°C, avec 6xSSC et le rinçage à T=55°C, avec 6xSSC et dans laquelle les conditions difficiles d'hybridation comprennent l'hybridation à T=65°C, avec 6xSSC et le rinçage avec 0.2xSSC à T=65°C.

19. Séquence d'acide nucléique isolé ou recombinant codant une protéine de CRG et étant capable d'hybrider une séquence d'acide nucléique selon la revendication 18 dans des conditions difficiles d'hybridation.

20. Séquence d'acide nucléique isolé ou recombinant selon l'une quelconque des revendications 16 à 19, dans laquelle la séquence d'acide nucléique code une séquence de consensus telle que définie dans la revendication 1 ou 2.

21. Séquence d'acide nucléique isolé ou recombinant selon la revendication 20, dans laquelle la séquence d'acide nucléique code les acides aminés 21 à 94 de la séquence d'acide aminé de la séquence n°2.

22. Partie de la séquence d'acide nucléique isolé ou recombinant selon la séquence n° 3, dans laquelle la partie comprend la séquence de promotion du gène codant la protéine de hCRG de la séquence d'acide aminé n°2, c'est-à-dire la séquence d'acide nucléique du nucléotide 1 jusqu'au premier nucléotide de l'exon 1 selon la séquence n°1, c'est-à-dire la partie entre les nucléotides 1 à 495 (= site Apa I) de la séquence n°3.

23. Procédé pour obtenir un anticorps spécifique pour un marqueur d'épithélioma spinocellulaire et d'épithélioma de cellules vésicales dans un échantillon ne comprenant normalement pas de kératinocytes ou de cellules épithéliales, ledit procédé comprenant l'utilisation d'une protéine de CRG isolé ou recombinant telle que définie dans les revendications 1 à 5 en tant qu'immunogène pour la production d'anticorps dans un animal de test et l'obtention de l'anticorps ainsi produit par l'animal de test.

24. Procédé selon la revendication 23 comprenant en outre l'étape consistant à fusionner les cellules qui produisent des anticorps par rapport à une protéine de CRG telle que définie dans les revendications 1 à 5 avec des cellules cancéreuses afin de générer des cellules d'hybridomes.
